# EUROPEAN PATENT SPECIFICATION

(11) **EP 1 617 884 B1**
(45) Date of publication and mention of the grant of the patent: **05.10.2011**
(21) Application number: 04750829.6
(22) Date of filing: 28.04.2004
(51) Int. Cl.: A61M 25/00, A61F 2/01, A61B 19/00

(54) **SLIDABLE CAPTURE CATHETER**
VERSCHIEBBARER FANGKATHETER
CATHETER DE CAPTURE COULISSANT

(30) Priority: 30.04.2003 US 466892 P; 27.04.2004 US 832565
(43) Date of publication of application: 25.01.2006
(73) Proprietor: Rubicon Medical, Inc., Salt Lake City, UT 84104 (US)
(72) Inventor: SWAINSTON, Kyle, W., Draper, UT 84020 (US); LINDER, Richard, J., Sandy, UT 84093 (US); EDMISTON, Daryl, R., Sandy, UT 84070 (US)
(74) Representative: Vossius & Partner
(86) International application number: PCT/US2004/013122
(87) International publication number: WO 2004/098674

(56) References cited:
- US-A- 5 098 440
- US-A- 5 944 728
- US-A- 6 152 946
- US-A1- 2002 120 286
- US-A1- 2002 121 472
- US-A1- 2002 128 678
- US-B1- 6 241 738
- US-B1- 6 383 193

## Description

### 1. The Field of the Invention

The present invention relates to devices for retrieving embolic protection devices in vascular vessels. In particular, the present invention relates to a catheter having a capture mechanism that can be actuated to capture and to retrieve a filter-type embolic protection device.

### 2. The Relevant Technology

Human blood vessels often become occluded or blocked by plaque, thrombi, other deposits, or material that reduce the blood carrying capacity of the vessel. Should the blockage occur at a critical place in the circulatory system, serious and permanent injury, and even death, can occur. To prevent this, some form of medical intervention is usually performed when significant occlusion is detected.

Several procedures are now used to open these stenosed or occluded blood vessels in a patient caused by the deposit of plaque or other material on the walls of the blood vessels. Angioplasty, for example, is a widely known procedure wherein an inflatable balloon is introduced into the occluded region. The balloon is inflated, dilating the occlusion, and thereby increasing the intraluminal diameter.

Another procedure is atherectomy. During atherectomy, a catheter is inserted into a narrowed artery to remove the matter occluding or narrowing the artery, i.e., fatty material. The catheter includes a rotating blade or cutter disposed in the tip thereof. Also located at the tip are an aperture and a balloon disposed on the opposite side of the catheter tip from the aperture. As the tip is placed in close proximity to the fatty material, the balloon is inflated to force the aperture into contact with the fatty material. When the blade is rotated, portions of the fatty material are shaved off and retained within the interior lumen of the catheter. This process is repeated until a sufficient amount of fatty material is removed and substantially normal blood flow is resumed.

In another procedure, stenosis within arteries and other blood vessels is treated by permanently or temporarily introducing a stent into the stenosed region to open the lumen of the vessel. The stent typically includes a substantially cylindrical tube or mesh sleeve made from such materials as stainless steel or nitinol. The design of the material permits the diameter of the stent to be radially expanded, while still providing sufficient rigidity such that the stent maintains its shape once it has been enlarged to a desired size.

Unfortunately, such percutaneous interventional procedures, i.e., angioplasty, atherectomy, and stenting, often dislodge material from the vessel walls. This dislodged material can enter the bloodstream, and can be large enough to occlude smaller downstream vessels, potentially blocking blood flow to tissue. The resulting ischemia poses a serious threat to the health or life of a patient if the blockage occurs in critical tissue, such as the heart, lungs, kidneys, or brain, resulting in a stroke or infarction.

To reduce the possibility of blockage, existing surgical procedures utilize an embolic protection device, such as a filter, which prevents passage of the embolic material dislodged during the percutaneous interventional procedure. Once the procedure is performed, the collected material must be removed without damaging the tissue of the patient or any device implanted within the patient, such as a stent. For instance, following deploying the filter and placing the stent, the filter must be removed without imposing any undue trauma on the vessel. Furthermore, the filter must be removed without letting any of the debris escaping into the vessel. That is, the filter must be able to retain all of the filter material.

Catheters have been developed to remove the filter and the contained debris. Typically, these catheters, usually termed "capture catheters" have a lumen that is sufficient to receive a deployed embolic protection device, such as a filter. Unfortunately, the outside diameter of such existing capture catheters is large relative to the body-lumen within which it is inserted. Where a stent has been placed in the vessel, these existing capture catheters can hit the stent, possibly dislodge the stent from the vessel, or, even worse, be blocked altogether from capturing the filter by the stent. Other capture catheters alleviate the above problems by using a smaller diameter distal end. However, the distal end of the capture catheter may not be large enough to capture the filter without causing collected emboli be squeezed through the pores of the filter, typically called "toothpasting." These flowing emboli can be dislodged from the filter by the catheter resulting in adverse effects to the patent.

Other attempts have been made to achieve a capture catheter that has a resilient distal end small enough to pass through the portion of the vessel having the stent, while being large enough to capture the filter which spans the entire transverse cross section of the vessel. However, the resilient material forming the distal end of the capture catheter is often elastically biased toward a smaller diameter. Thus, when the filter is captured by the catheter, the material in the filter is compressed. Therefore, the emboli have a tendency to "toothpaste" out of the capture catheter. Thus, it would be an advantage to provide a capture catheter which has a distal end small enough to pass through already deployed stents in the vessel, but large enough to adequately capture the filter or other embolic protection device.

Another problem with existing capture catheters is that an operator typically has to use both hands to operate the capture catheter and collect the embolic protection device. For instance, the operator uses one hand to maneuver the catheter into position while keeping a guide wire or shaft associated with the embolic protection device steady. The other hand is used to exchange a capture mechanism or assembly that retrieves the filter. That is, both hands are required to deploy the capture mechanism. It would thus be an advantage to provide a capture catheter which allows an operator to use the capture catheter in a simpler single-handed manner that provides more precise control of the capture catheter and the guide wire.

Furthermore, most existing capture catheters do not provide a mechanism to stop a distal end of the capture catheter in the proximal or distal direction, preventing overreaching. It would thus be an advantage to have a capture catheter that makes it easier for an operator to detect when the capture catheter has been correctly placed and when the capture mechanism should be deployed. Furthermore, it would be an advantage to have a capture catheter that prevents overreaching by the capture mechanism.

In addition, in some existing capture catheters, it is difficult to sense when the catheter is in close proximity to the filter or when the operator can receive the filter. That is, there is a lack of tactile feel in most existing capture catheters.

Finally, most capture catheters have the capture mechanism disposed at the distal-most end. Generally, the capture mechanism is a relatively large mechanism as it must be large enough to capture the filter. Thus, when the capture mechanism is inserted directly into the vascular vessel, such a larse-diametered object can cause undue trauma to the vessel.

Methods and devices for delivering and/or retrieving a filter fixed to a guidewire are disclosed in US 2002/0128678, which relates to a system comprising an outer shaft having a proximal end, a distal end and a wall defining an outer shaft lumen. The system also includes a stop mechanism disposed within the outer shaft lumen. The stop mechanism is preferably configured such that relative axial movement between the guidewire and the outer shaft may be selectively precluded.

In view of the above, it would be an advantage to provide a capture catheter with a distal end small enough to prevent undue trauma in the vessel, but with a large enough capture mechanism to efficiently and effectively capture the filter.

### BRIEF SUMMARY OF THE INVENTION

The present invention provides capture catheters having the features defined by the claims.

The capture catheters of the present invention are configured to capture an embolic protection device that has collected embolic material during a surgical procedures. Usually, the embolic protection device, such as a filter, can be connected to the end of a guide wire. The capture catheter of the present invention can include a proximal end and a distal end. A tubular member extends between the proximal and distal ends of the capture catheter. At the distal end of the capture catheter can be a capture sleeve. An actuation assembly mounts to the proximal end of the capture catheter and can be configured to deploy the capture sleeve to capture a filter upon activating the actuation assembly.

In one illustrative configuration, the actuation assembly can include a barrel, a plunger and a deploying member. The proximal end of the tubular member receives the barrel and the barrel receives the plunger so that the barrel guides the plunger in a linear fashion. Annular lips at the proximal and distal ends of the barrel can cooperate with corresponding components in the plunger to provide proximal and distal mechanical stops. In addition to mechanical stops, the barrel can provide outer markings to indicate maximum proximal and distal locations.

The proximal end of the deploying member can be connected to the plunger. The plunger can include means for reinforcing the proximal end of the deploying member. For example, the plunger can include outer and inner telescoping members that are spaced apart to receive the tubular member. The inner telescoping member connects or couples to the proximal end of the deploying member to reinforce the wire.

In one illustrative configuration, the tubular member can having a dual lumen configuration, with a first lumen receiving a deploying member movable by the actuation assembly and a second lumen receiving the guide wire. The guide wire lumen can extend along the entire length of the tubular member or can extend only partially along the length thereof. For instance, the second lumen can extend from a distal end toward a proximal end of the capture catheter and terminate distal to the proximal end of the capture catheter.

The distal end of the deploying member can be connected to the capture sleeve. In one embodiment_{,} a connecting member can be used to connect the deploying member to the capture sleeve. The connecting member can be a ring, disposed around the inner circumference of the capture sleeve. The connecting member in conjunction with the deploying member allows the capture sleeve to be displaced proximally and distally. The distal end of the deploying member can also be reinforced by means for reinforcing the proximal end of the deploying member.

In an illustrative embodiment, a secondary sleeve or follower sleeve can extend from the capture sleeve toward the proximal end of the capture catheter. This sleeve extends over the interface between the tubular member and the capture sleeve. The follower sleeve protects the deploying member and acts as a transition between the tubular member and the capture sleeve. In another embodiment, the proximal portion of the capture sleeve can be elongated to cover the portion of the distal end of the tubular member. Follower sleeve and/or capture sleeve can be configured in conjunction with tubular member to form mechanical, incremental stops.

Formed at a distal end of the tubular member can be a distal tip. This distal tip can be connected to the tubular member by a spacer member, although the tip can be integrally formed with the tubular member. The capture sleeve can be slidably disposed over the distal tip, with the distal tip providing a small-bored tip which reduces the trauma when inserting the capture catheter in the vascular vessel. Furthermore, the distal tip can act as a transition between the small-bored tip and the larger-sized bore of the capture sleeve. In addition, the distal tip, spacer member and tubular member can cooperate to provide a mechanical stop in the proximal and distal directions.

In one embodiment, the capture sleeve includes a restraining member cooperating with a reinforcement mechanism. The restraining member can be a thin-walled sleeve and have sufficient elasticity and resiliency to aid with capturing the deployed embolic protection device. Further, the restraining member can be a mesh or have a uniform thickness. The reinforcement mechanism can include one or more wires that are embedded in the restraining member. Alternatively, the one or more wires could be disposed on the outside or inside of the restraining member. The one or more wires could be formed in various configurations such as a braided configuration, a coiled configuration or a mesh configuration.

In operation of an illustrative capture catheter, the capture catheter is prepared to be inserted into the vascular vessel. That is, the plunger and barrel are positioned in the fully loaded position in which the plunger is disposed proximally in the barrel. As such, the capture sleeve can be positioned in the proximal-most position. The air can be aspirated from the tubular member through an optional flush port and/or through the second lumen. The capture catheter can be inserted into the vascular system using the guide wire to direct and navigate the capture catheter as it is maneuvered in the vascular system.

When the capture catheter has reached the filter location, the operator activates the actuation assembly. This can involve pressing the plunger distally in the barrel to displace the deploying member in a distal direction. In turn, the capture sleeve is displaced distally to at least partially enclose the filter having the embolic debris disposed therein. In the event that an inaccurate or incomplete capture occurs, the operator can move the plunger of the actuation assembly to release the filter. The operator can then attempt to recapture the filter.

Once the capture sleeve has been deployed, the operator can then lock the actuation assembly in the deployed position and remove the capture catheter, including the guide wire, from the vascular system. Alternatively, the operator can use the same process to reposition the filter, i.e., activate the capture sleeve to capture the filter, lock the actuation assembly, move the filter and capture catheter, and release the capture catheter to release the filter.

The present invention thus provides a capture catheter with distal tip with a small bore and a capture sleeve having a larger bore. The present invention provides a two-phase profile for the capture catheter which allows the catheter to pass through stents or other apparatuses deployed in the vessel but large enough to capture the filter and the embolic debris contained therein without dislodging either the stent or the debris in the filter. The present invention, therefore, provides a simple sliding mechanism which eliminates the need for the operator to actually handle the deploying member, the movement of the plunger being translated through the deploying member to the capture sleeve.

These and other objects and features of the present invention will become more fully apparent from the following description and appended claims, or can be learned by the practice of the invention as set forth hereinafter.

### BRIEF DESCRIPTION OF THE DRAWINGS

To further clarify the above and other advantages and features of the present invention, a more particular description of the invention will be rendered by reference to specific embodiments thereof which are illustrated in the appended drawings. It is appreciated that these drawings depict only typical embodiments of the invention and are therefore not to be considered limiting of its scope. The invention will be described and explained with additional specificity and detail through the use of the accompanying drawings in which:
Figure 1 illustrates a perspective view of one embodiment of a capture catheter in accordance with the present invention;
Figure 2 illustrates a cross-sectional view of the capture catheter of Figure 1;
Figure 3 illustrates a cross-sectional view of the capture catheter of Figure 1;
Figure 4 illustrates a cross-sectional view of another embodiment of a capture catheter in accordance with the present invention;
Figure 5A illustrates a cross-sectional view of an embodiment of the capture sleeve in accordance with the present invention;
Figure 5B illustrates a top plan view of another embodiment of the capture sleeve;
Figure 5C illustrates a top plan view of yet another embodiment of the capture sleeve;
Figure 5D illustrates a cross-sectional view of the capture sleeve of Figure 5C;
Figure 6A illustrates a method of using the capture catheters of the present invention, illustrating the capture sleeve in a non-deployed state;
Figure 6B illustrates a method of using the capture catheters of the present invention, illustrating the capture sleeve in a deployed state;
Figure 7 illustrates another embodiment of the capture catheter of the present invention, where a control wire is attached to a control wire tubular member;
Figure 8 illustrates another embodiment of the capture catheter of the present invention, where a control wire is inserted into the capture sleeve;
Figure 9 illustrates another embodiment of the capture catheter of the present invention, where a distal tip includes a distal member;
Figures 10A-10C illustrate alternate embodiments of the capture catheter and the transition between portions thereof;
Figure 11 illustrates another embodiment of the capture catheter of the present invention, where a control wire and capture sleeve are installed over the tubular member and distal member assembly; and
Figure 12 illustrates the capture catheter of Figure 11 completely assembled with the capture sleeve extended.
Figure 13 - 18

### DETAILED DESCRIPTION OF THE EXEMPLARY EMBODIMENTS

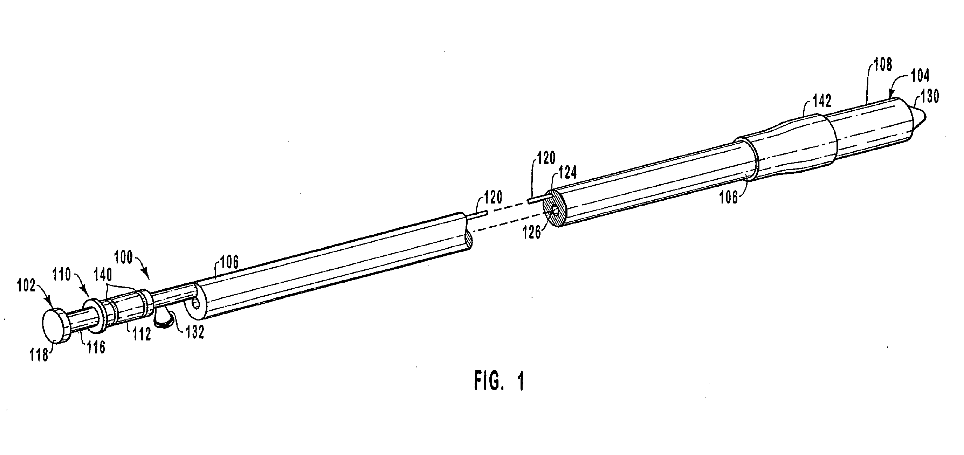

With reference to Figure 1, illustrated is one exemplary capture catheter 100. In general, capture catheter 100 can be used to capture an embolic protection device that collects embolic material during an interventional procedure. Usually, the embolic protection device, such as, but not limited to, a filter is connected to the end of a guide wire. As used herein, the term "guide wire" can refer to any wire or hypo-tube that can function as a guide wire, i.e., steerable through the tortuous anatomy of a patient, allow other medical devices to be exchanged over the same, etc. The features and components of the present invention are not necessarily limited to capture catheters but can apply to other vascular procedures and devices.

Generally, the capture catheter of the present invention eliminates the problems associated with accessing and positioning the capture catheter and effectively capturing a deployed embolic protection device. For instance, the capture catheter can have a diameter that is sufficiently small to allow at least a portion of the catheter to pass through a vascular vessel and a stent or other obstruction in the vascular vessel. The capture catheter can have a capture sleeve that is sufficiently elastic to capture the embolic protection device, while being sufficiently rigid to prevent buckling or crumpling when brought into contact with the embolic protection device. The capture sleeve can also be sufficiently resilient to retain the embolic material captured by the embolic protection device, while reducing the possibility that the capture catheter applies a force sufficient to cause squeezing or "tooth-pasting" of the embolic material form the interior of the embolic protection device.

With continued reference to Figure 1, capture catheter 100 includes a proximal end 102 and a distal end 104. A tubular member 106 extends between proximal end 102 and distal end 104. Disposed at distal end 104 of capture catheter 100 is a capture sleeve 108, while an actuation assembly 110 is disposed at proximal end 102. The actuation assembly 110 selectively displace capture sleeve 108 in the proximal and/or distal directions.

In the illustrated configuration of Figure 1, the actuation assembly 110 includes a barrel 112 that slidably receives a plunger 116. In this sense, barrel 112 can form a housing for plunger 116. Moving plunger 116 relative to barrel 112 causes a deploying member 120 to move relative to tubular member 106. Moving plunger 116 relative to barrel 112 moves deploying member 120 relative to tubular member 106 and so actuates the capture mechanism 108 disposed at distal end. The control provided by the barrel/plunger configuration aids with accurately and controllably positioning capture mechanism 108 in engagement with the embolic protection device (not shown) during the process of capturing the embolic protection device (not shown).

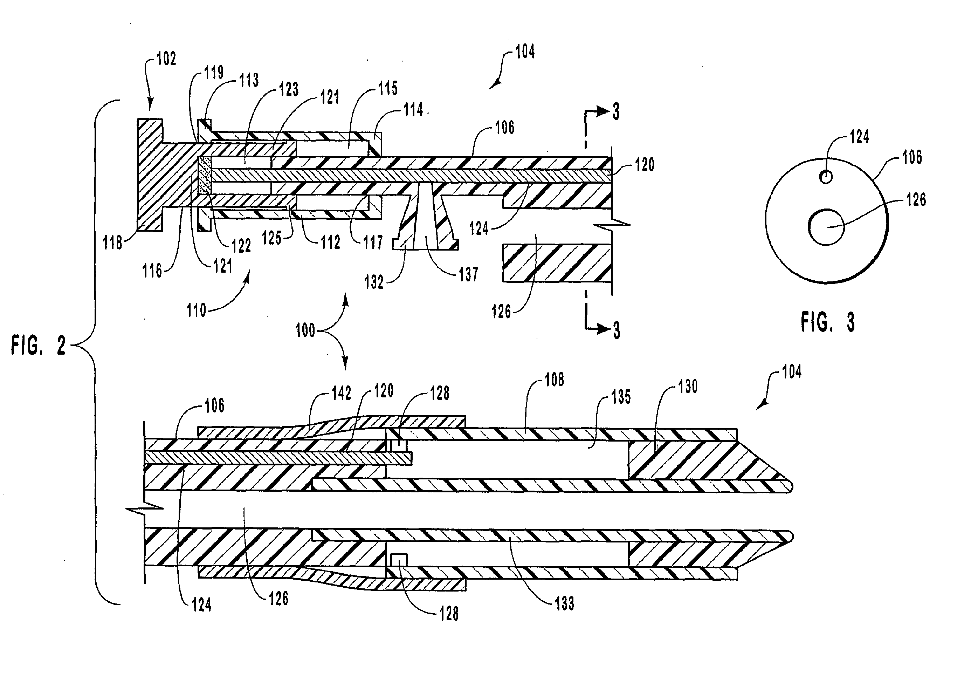

With reference now to Figure 2, barrel 112 of actuation assembly 110 is disposed at the proximal end of tubular member 106. Barrel 112 has a proximal end 113, a distal end 114, and a chamber 115 extending from proximal end 113 to distal end 114. The chamber 115 receives plunger 116 and so has a complementary configuration to at least a portion of plunger 116. The distal end 114 of barrel 112 has an aperture 117 that cooperates with tubular member 106 so that tubular member 106 can be securely or removably disposed within aperture 117. For instance, tubular member 106 can be attached to barrel 112 through use of adhesives, composite adhesives, complementary threads, slip-fit, friction fit, or interference fit bonds, or various other techniques, such as but not limited to, welding, soldering, thermal bonding, chemical bonding, or other techniques that facilitate securely or releasably disposing a tubular member within an aperture.

At proximal end 113, barrel 112 has an optional annular lip 119 disposed inwardly to plunger 116, when plunger 116 is disposed within chamber 115. As such, barrel 112 guides plunger 116 in a linear fashion. Plunger 116 includes a shaft 121 having a head 118 attached to the proximal end thereof. Head 118 can have a larger cross-section than shaft 121 such that when fully distally disposed in barrel 112, head 118 may rest on a proximal end of barrel 112. It will be appreciated that plunger 116 is slidably disposed in barrel 112 so that it can be selectively operated in the proximal and/or distal directions. Plunger 116 further includes an annular bore or recess 123. Annular recess 123 can be adapted to receive the distal end of tubular member 106. Plunger 116 is able to slide over the distal end of tubular member 106 while being guided by barrel 112. In other configuration, plunger 116 slides within chamber 115, with a proximal end of tubular member 106 terminating at aperture 117 and deploying member 120 attaching to plunger 116. Therefore, plunger 116 may or may not cooperate with tubular member 106.

Plunger 116 is able to be displaced in variable positions along barrel 112 between a fully proximal and a fully distal position. The maximum proximal and/or distal positions can be determined by stops. Proximal stops can be provided by forming an inward annular lip at the proximal end 114 of barrel 112 and forming an outward annular lip 125 on the distal end of plunger 116. When the annular lips meet, plunger 116 is prevented from being removed from barrel 112. Distal stops can be provided in at least two locations. Distal end 114 of barrel 112 prevents the distal end of plunger 116 from sliding distally past aperture 117. In addition, the proximal end of barrel 112 in combination with head 118 form a distal stop when head 118 rests against the proximal end of barrel 112. These stops can be used to reference the operator between starting and stopping points during the capture procedure.

One skilled in the art will appreciate that various other configurations of stops are capable of performing the desired function of preventing excessive movement of the plunger relative to the barrel, whether such movement is in the proximal direction or the distal direction. For instance, embodiments of the activation assembly can be devoid of stops, while relying upon the skill of the operator to control the displacement of the plunger relative to the barrel.

In still another configuration, an inner surface of barrel 112 and a portion of plunger 116 can have complementarily engaging structures. As the physician or clinician moves plunger 116 with barrel 112, the structures engage to provide a tactile feel to the position of the distal end 104 of catheter 100. The engagement of these structures, such as but not limited to, recesses, stops, protrusions, or other structures, may be overcome by additional force being exerted by the physician or clinician.

Alternatively or in addition to mechanical stops, barrel 112 provides reference points for deploying the capture sleeve 108. In one embodiment, illustrated in Figure 1, barrel 112 is transparent and can have markers 140 on the outside thereof so that plunger 116 is visible therethrough. These markers 140 indicate the maximum and minimum movement of plunger 116 in barrel 112. As plunger 116 is pushed through barrel 112, the distal end of plunger 116 can be viewed in relation to markers 140 on barrel 112 so that the operator is aware of the exact position of capture sleeve 108.

As plunger 116 is moved within barrel 112, the frictional contact between plunger 116 and chamber 115 is sufficient to prevent inadvertent movement of plunger 116 relative to barrel 112. This friction interface between plunger 116 and barrel 112 can be considered a locking mechanism. In other configuration, actuation assembly 100 can have a separate locking mechanism which fixes plunger 116 in relation to barrel 112 until the lock is released by the operator. It will be appreciated that a variety of locking mechanisms can be implemented to provide a lock between plunger 116 and barrel 112. For instance, threads formed within the chamber and upon the surface of the plunger can mate to lock the plunger relative to the barrel. In another configuration, elements extending from the plunger can engage with a proximal end of the barrel to prevent movement of the plunger relative to the barrel. Various other locking mechanisms are known to those skilled in the art.

With continued reference to Figure 2, movement of plunger 116 within barrel 112 causes movement of deploying member 120, which may be a component of actuation assembly 110. In the embodiment of Figure 2, deploying member 120 is configured as a wire. The proximal end of deploying member 120 is connected to plunger 116, which the distal end of deploying member 120 connects to capture sleeve 108. Although reference is made to deploying member 120 being a wire, it will be understood that deploying member 120 can be a fiber, cord, rod, shaft, or other sufficiently stiff structure to translate movement along the length of capture catheter 100. Deploying member 120 can be stainless steel, shaped memory metal, or alloys, a rigid plastic, a rigid polymer, synthetic material, or other material so long as it is sufficiently stiff enough to displace capture sleeve 108.

The proximal end of deploying member 120 is connected to plunger 116 by a connecting element 122 disposed in annular recess 123. In one configuration, connecting element 122 can be a glue ball bond joint formed from a polymer or composite, a metal, a plastic, or the like and configured to hold the proximal end of deploying member 120. Connecting element 122 can be disposed in annular recess 123 by interference fit, threaded connection, adhesive, welding, soldering, laser welding, snap fit, and the like. In another configuration, connecting element 122 can be a swivel connection so that deploying member 120 can rotate in relation to plunger 116. In still another configuration, deploying member 120 can mount directly to plunger 116 with the use of recess 123 and/or connecting element 122. Rather, deploying member 120 can interference fit, threadably connect, or otherwise connect to any portion of plunger 116.

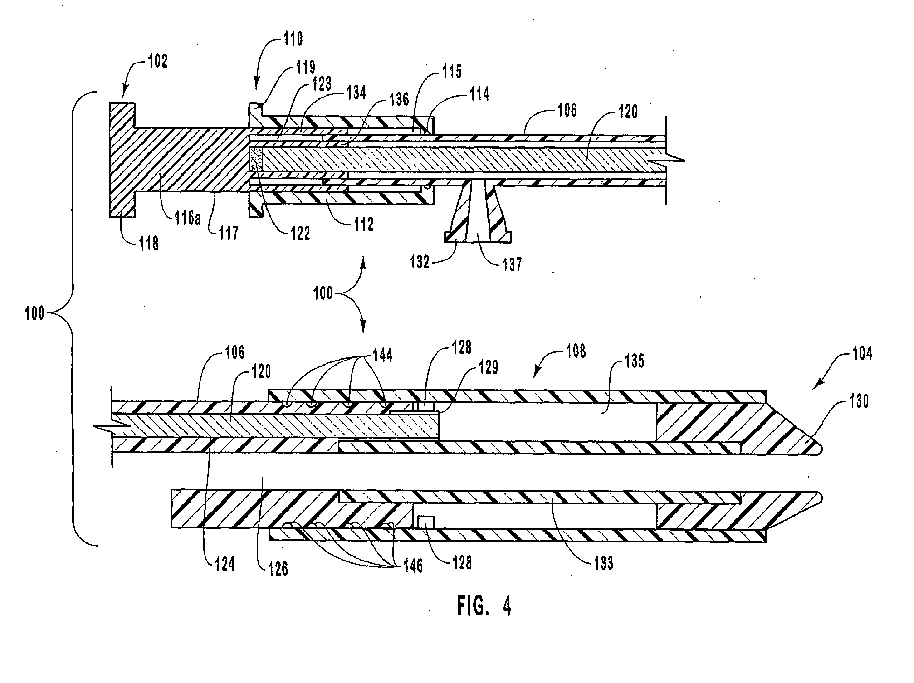

Turning to Figure 4, plunger 116 can have an alternative embodiment to help reinforce the proximal end of deploying member 120. Because deploying member 120 is very thin it may bend or buckle when plunger 116 exerts force against the proximal end thereof. The alternate plunger, identified by reference number 116a can optionally include an outer telescoping member 134 and inner telescoping member 136. Inner telescoping member 136 is in close proximity with deploying member 120. Outer telescoping member 134 and inner telescoping member 136 can be formed as distinct parts, for example, sleeves, which are bonded to the proximal end of deploying member 120 by adhesive, epoxy, crimping, and the like. The proximal ends of outer and inner telescoping members 134, 136 are then connected to plunger 116a by bonding techniques known in the art. Members 134, 136 can alternatively be formed integrally with plunger 116a.

Inner telescoping member 136 is bonded to the proximal end of deploying member 120 by friction fit, threaded connection, adhesive, welding, soldering, laser welding, snap fit, interference fit, or other techniques or manners identified herein, or those understood by those of skill in the art. As such, inner telescoping member 136 supports the proximal end of deploying member 120 so that it does not buckle or collapse during deployment. Alternatively, the proximal end of deploying member 120 can be crimped and then bonded to inner telescoping member 136. Outer and inner telescoping members 134, 136 can be coaxially aligned and spaced apart to be slidably disposed with the proximal end of tubular member 106. Thus, in this alternative embodiment, the proximal end of deploying member 120 is reinforced.

Referring back to Figure 2, deploying member 120 can be disposed in an actuation lumen 124 formed along the length of tubular member 106. As such, deploying member 120 extends between plunger 116 and distal end 104 of capture catheter 100. The size of actuation lumen 124 is in close proximity to that of deploying member 120 so actuation lumen 124 reinforces or supports deploying member 120 throughout the length thereof. Thus, distal or proximal movement of plunger 116 is translates to deploying member 120 which, in turn, translates to movement of capture sleeve 108. The actuation assembly 110 thus provides a remotely actuated capture mechanism.

Tubular member 106 can be configured to have a dual-lumen configuration along at least a portion of its length, a cross-section of which is illustrated in Figure 3. Thus, in addition to the actuation lumen 124, tubular member 106 also includes a guide wire lumen 126. Guide wire lumen 126 receives the guide wire having the filter assembly (not shown) connected thereto. Tubular member 106 can be constructed of any material allowing extrusion such as, but not limited to, stainless steel, other biocompatible metals, alloys, polymers, plastics, synthetics, composites, and the like. In one embodiment, at least a portion of tubular member 106 is formed from a polyimide material. Tubular member 106 and one or both of lumens 124 and/or 126 can also be Teflon-coated.

In embodiments using stainless steel or other metal, tubular member 106 can be formed having two tubular members bonded together by welding, adhesive or other bonding or coupling techniques known to those skilled in the art. Alternatively, tubular member 106 could include a single metal tube having a pair of lumens formed therein. In those embodiments using a plastic or polymer composition, tubular member 106 can be either formed by any known extrusion process in order to simultaneously form actuation lumen 124 and guide wire lumen 126 or can be formed from two or more tubular members joined together. In one embodiment, the polymeric material is a thermoplastic, which allows for tubular member 106 to be extruded. It is also possible for tubular member 106 to be fabricated from two separate, synthetic tubular members that are joined together using appropriate techniques for the synthetic material used.

Tubular member 106 does not have to be the same material throughout its entire length. In one embodiment, the distal portion of tubular member 106 includes a polymeric material while the rest of the tubular member 106 includes stainless steel. This allows tubular member 106 to have sufficient rigidity to be maneuvered and pushed through vascular vessels. In addition, the distal polymeric portion provides a certain elasticity that assists tubular member 106 to be inserted through curved portions of the vasculature as it follows or tracks the guide wire. The distinct parts of tubular member 106 can be connected or bonded by techniques known in the art such as, but not limited to, adhesive, welding, soldering, laser welding, epoxy, and the like.

Actuation lumen 124 and guide wire lumen 126 can be sized to be in close proximity to the respective wires disposed therein. That is, actuation lumen 124 and guide wire lumen 126 have a diameter slightly larger than the wire disposed therein such that the wire is able to slide through the lumen in the proximal and/or distal directions. Particularly with regard to the actuation lumen 124, because deploying member 120 is so small in diameter, actuation lumen 124 serves to reinforce deploying member 120 along the length of tubular member 106 so that deploying member 120 does not collapse or buckle at any point along the length thereof.

The dual-lumen configuration discussed above can extend along the entire length of tubular member 106 or can extend only partially along the length of tubular member 106. That is, guide wire lumen 126 can only extend a partial length of tubular member 106, leaving actuation lumen 124 to extend along the entire length of tubular member 106. For example, in the embodiment of Figure 2, tubular member 106 is configured as a "full exchange" tubular member. That is, both deploying member lumen 124 and guide wire lumen 126 extend substantially the entire length of tubular member 106. Thus, a guide wire is configured to pass through substantially the entire length of tubular member 106 before exiting therefrom.

In another embodiment, illustrated in Figure 4, tubular member 106 is configured as a "rapid exchange" tubular member. That is, deploying member lumen 124 extends along the entire length of tubular member 106 while guide wire lumen 126 extends only along a partial length of tubular member 106. Thus, a guide wire passes through only a portion of tubular member 106 before exiting therefrom. As shown in Figure 4, the guide wire exits near the distal end of tubular member 106. In one particular embodiment having a rapid exchange configuration, the distal thirty centimeters of tubular member 106 includes a dual-lumen configuration while the remainder of tubular member 106 proximal that portion includes a single-lumen configuration having only the deploying member lumen 124. In this configuration, tubular member 106 can be formed from a single lumen member that is connected to a dual lumen member, with the dual lumen member having both a lumen for receiving the guide wire and a lumen for receiving the deploying member. It will be understood that a lesser or greater length of the distal end of the tubular member 106 can include the dual lumen. In one embodiment, a length of about 3 cm, in another embodiment, a length of about 170 cm, and in another embodiment, a length between about 3 cm and about 170 cm. Generally, the tubular member 106 can have a length selected form the type of delivery techniques, so that lengths of greater or lesser than thirty centimeters are possible.

Referring back to Figure 2, the distal end of deploying member 120 is connected to capture sleeve 108. Deploying member 120 can be connected to capture sleeve 108 by any manner or technique which provides sufficient connection strength between deploying member 120 and sleeve 108 so that they do not separate and deploying member 120 is able to translate movement to capture sleeve 108. In one embodiment, deploying member 120 can be connected directly to capture sleeve 108 by adhesive, bonding, or other manners or techniques that facilitate connecting one member to another member. However, it may be desirable to maintain deploying member 120 substantially parallel or as parallel as possible to capture sleeve 108 to facilitate translation of movement. As such, as shown in Figure 2, deploying member 120 is connected to capture sleeve 108 through a connecting member 128.

In one embodiment, connecting member 128 is a ring having a diameter slightly smaller than capture sleeve 108 so that it fits on the inside thereof. However, connecting member 128 can be any structure that provides sufficient connection strength between deploying member 120 and capture sleeve 108. Other illustrative configurations for connecting member 128 are discussed in more detail below.

Connecting member 128 is rigidly connected to the proximal end of capture sleeve 108. As shown in Figure 2, connecting member 128 is disposed at the proximal end of capture sleeve 108. Connecting member 128 can be constructed of any material that is sufficiently stiff to be able to slide capture sleeve 108 in the proximal and/or distal directions. In one embodiment, connecting member 128 is constructed of steel. In other embodiments, connecting member 128 can be constructed of plastic, polymer, silicon, composite materials, metals, metal alloys, synthetics, and the like.

Connecting member 128 is connected to capture sleeve 108 by any manner known in the art, such as, but not limited to, welding, adhesive, soldering, laser welding, and the like. Similarly, deploying member 120 is connected to connecting member 128 by welding, adhesive, soldering, laser welding, or other technique for bonding one member to another member. In view of the foregoing, it will be appreciated that capture sleeve 108 can slide distally or proximally depending upon how actuation assembly 110 is operated.

With reference to Figure 4, the distal end of deploying member 120 can optionally include a reinforcement member 129 that is disposed over the end thereof. The reinforcement member 129 is shown as a sleeve or tube disposed about the distal end of deploying member 120. Reinforcement member 129 can be constructed of a metal, alloy, synthetic, composite, or polymeric material, such as polyester. Reinforcement member 129 can be crimped or bonded to deploying member 120. Bonding can be by any manner known in the art such as by welding, adhesive, soldering, laser welding, or other technique for bonding or attaching two components together. Reinforcement member 129 is then connected to connecting member 128 by appropriate technique or manner, such as those described herein and such other understood by one skilled in the art. Reinforcement member 129 helps keep deploying member 120 parallel to capture sleeve 108 so that as deploying member 120 is advanced distally, it does not buckle or collapse.

While reinforcement member 129 is shown as a singular sleeve-like member, it will be appreciated that reinforcement member 129 can be formed from multiple parts or even a wire coiled or braided around the distal end of deploying member 120. In addition, other mechanisms can be used to prevent deploying member 120 from buckling or collapsing at the distal end, such as a spring coil.

In the embodiment of Figure 2, the proximal end of capture sleeve 108 does not overlap tubular member 106. When capture sleeve 108 is displaced in the distal direction, deploying member 120 becomes exposed. When the distal end 104 of capture catheter 100 is in a curved portion of the vessel, or even in a straight portion, even a little resistance could cause deploying member 120 to bow or bend, making it difficult to deploy capture sleeve 108. Thus, a secondary or follower sleeve 142 is positioned over the transition of tubular member 106 and capture sleeve 108. The distal portion of follower sleeve 142 connects to capture sleeve 108. The follower sleeve 142 provides at least two functions: (1) protecting deploying member 120 to allow it to continue to translate motion in an axial direction; and (2) providing a smooth transition between tubular member 106 and capture sleeve 108.

In another embodiment illustrated in Figure 4, secondary or follower sleeve 142 can be formed integrally with capture sleeve 108. Stated another way, the proximal portion of capture sleeve 108 is elongated to allow it to cover the interface between tubular member 106 and capture sleeve 108. The capture sleeve 108 overlaps the end of tubular member 106. Capture sleeve 108 is slidably disposed over tubular member 106. In this embodiment, connecting member 128 is positioned between a proximal end and a distal end of capture sleeve 108. Thus, as capture sleeve 108 is distally displaced by deploying member 120, deploying member 120 remains protected by the proximal portion of capture sleeve 108.

Referring back to Figure 2, extending from guide wire lumen 126 is a spacer member 133. Spacer member 133 can be constructed of a semi-resilient material such as rubber, plastic, synthetic material, and the like. The spacer member 133 provides a channel or lumen through which the guidewire (not shown) can pass during use of capture catheter 100. Thus, guide wire lumen 126 extends from distal end 104 of capture catheter 100 toward proximal end 102, whether or not lumen 126 extends completely or partially to proximal end 102. Although reference is made to the inclusion of spacer member 133, one skilled in the art will understand that a portion of tubular member 106 may extend to a distal end of capture catheter 100.

Disposed at a distal end of spacer member 133 is a distal tip or bushing 130. The distal tip 130 acts as a transition between the outside diameters of the guidewire disposed within guide wire lumen 126 and capture catheter 100. In this regards, tip 130 includes a tapered distal end that acts as an atraumatic tip of capture catheter 100. This aids with maneuvering and navigating capture catheter 100 through the blood vessel.

Distal tip 130 also functions to prevent capture catheter 100 from passing over the filter when capture catheter 100 comes in contact with the filter. The lumen 126 extending through tip 130 is sufficiently small to prevent passage of capture catheter 100 over the embolic protection device to be captured by capture catheter 100. In addition to the above, distal tip 130 also functions to center capture sleeve 108 over the guide wire and provides a surface over which catheter assembly 108 can slide during deployment and thereof acts as a stop for distal movement of catheter assembly 108. Although reference is made to the inclusion of distal tip 133 mounted to spacer member, one skilled in the art will appreciate that a structure capable of performing the functions at distal tip 130 can be integrally formed with spacer member 133 or tubular member 106 when the tubular member 106 extends to a distal end of capture catheter 100.

Distal tip 130 can be constructed of a semi-resilient material such as rubber, plastic, synthetic material, and the like. In one embodiment, distal tip 130 is formed from polyimide tubing. Distal tip 130 can be formed by processes known in the art such as grinding the tip from a plastic or polymer extrusion, molding, injection molding, insert molding, or extruding the distal tip.

Distal tip 130 can also have fluoroscopic markings to assist the operator in viewing the location and placement of capture catheter 100. One or more of distal tip 130, spacer member 133, and tubular member 106 can be connected by any manner or technique known in the art such as, but not limited to, adhesive, welding, soldering, laser welding, and the like. Alternatively, one or more of distal tip 130, spacer member 133 and tubular member 106 can be extruded as an integral member.

Distal tip 130 and spacer member 133 also provide a mechanical stop in the proximal and/or distal directions to prevent overreaching or overextending of capture sleeve 108. As illustrated in Figures 2 and 4, because spacer member 133 has a smaller diameter than tubular member 106 and distal tip 130, a space or region 135 exists therebetween. Connecting member 128 can move within this space or region 135. The configuration of this region 135 or area can vary based upon the configurations of deploying member 120, capture sleeve 108, connecting member 128, and tubular member 106.

A proximal mechanical stop is provided by the interface between tubular member 106 and connecting member 128. In addition, a distal mechanical stop is provided when connecting member 128 abuts or comes in contact with distal tip 130. In one embodiment, the distance between tubular member 106 and distal tip 130 generally corresponds to the distance between on the maximum proximal placement and maximum distal displacement of plunger 116 within barrel 112, which also act as mechanical stops.

Furthermore, capture sleeve 108 and tubular member 106 can also provide means for incrementally stopping capture sleeve 108. As shown in Figure 4, the proximal end of capture sleeve 108 can include a stop member 144 in the form of a ring or a ridge member. The distal end of tubular member 106 can include a plurality of grooves 146 which are configured to receive stop member 144. Preferably, the depth of grooves 146 is not so great as to completely prevent capture sleeve 108 from moving in a distal or proximal direction. As actuation assembly 110 displaces capture sleeve 108 in the proximal direction, stop member 144 will rest in each groove 146 when it comes in contact with that particular groove. Deploying member 120 is sufficiently stiff so that movement of deployment member 120 displace stop member 144 from groove 146, allowing capture sleeve 108 to continue to displace distally or proximally as the case may be. However, when deploying member 120 is not applying force to capture sleeve 108, stop member 144 and groove 146 cooperate to form a mechanical, incremental stop. The operator will be able to tactically feel the incremental stops which can prove useful in deploying the capture sleeve 108.

Stop member 144 and groove 146 can assist in retaining capture sleeve 108 at a particular position. For example, once the operator has captured the filter, stop member 144 and groove 146 maintain capture sleeve 108 in place so that when the operator removes his or her hand from barrel 112 and plunger 116 or 116a, capture sleeve 108 does not displace back in the proximal direction, ensuring that the filter is retained therein. Barrel 112 and plunger 116 or 116a could also be configured with stop and grooves or other mechanisms to provide incremental stops. It will be appreciated that other ratcheting configurations can be applied to provide incremental stops in the proximal or distal end of capture catheter 100

In another embodiment, tubular member 106 can extend along the entire length of capture sleeve 108 to terminate with a tapered end, thus eliminating the need for distal tip 130 and spacer member 133. In this alternate embodiment, tubular member 106 would still have the dual-lumen configuration. Connecting member 128 would be in the form of a wedge or elongate rectangular piece rigidly connecting capture sleeve 108 to deploying member 120. A portion of tubular member 106 could be configured to slidably receive connecting member 128 and allow connecting member 128 to slide therethrough, allowing deploying member 120 to displace capture sleeve 108 in the proximal and/or distal direction. In this alternate embodiment, tubular member 106 can include a slot or region to receive a portion of connecting member 128. The slot or region of smaller diameter could be formed such that the proximal end of the slot or region provides a proximal mechanical stop and the distal end of the slot or region provides a distal mechanical stop. Capture sleeve 108 and tubular member 106 could also be configured to form incremental mechanical stops.

Referring back to Figure 2, a flush port 132 can be disposed at the proximal end of tubular member 106. Flush port 132 includes an aperture 137. Specifically, aperture 137 of flush port 132 can be disposed in communication with actuation lumen 124. Flush port 132 can be configured to allow a fluid such as saline or heparnized saline to be injected into actuation lumen 124. Flush port 132 allows an operator to aspirate or remove air from capture catheter 100 to prevent the possibility of injecting air into the vascular system. The fluid fills actuation lumen 124 throughout the entire tubular member 106. When fluid exits the distal end of tubular member 106, the operator is ensured that the air inside actuation lumen 124 has been evacuated. Flush port 132 can connect to a variety of different medical devices, such as but not limited to, a syringe to aspirate capture catheter 100. In one embodiment, flush port 132 is configured as a luer lock connection. Other structures that enable connecting of a medical device to capture catheter are contemplated, such as, but not limited to, threaded connection, snap-fit connection, friction fit connection, combinations thereof, or other structures capable of facilitating connecting a medical device to the capture catheter.

In one embodiment, barrel 112 and plunger 116 and flush port 132 are provided by modified syringe components. For example, a syringe barrel providing the barrel 112 and plunger 116 components can be connected to a lure lock T-connecter. The T-connector is connected to a lure hub which provides flush port 132. The lure hub is bonded with adhesive to a steel, stainless steel cylindrical hypo-tube shaft which provides tubular member 106, while a metallic shaft mounts to plunger 116 to act as deployment member 120. In another embodiment, illustrated in Figure 2, flush port 132 is formed integrally with tubular member 106 and barrel 112 is bonded or secured to the proximal end of tubular member 106. In yet another embodiment, barrel 112, tubular member 106 and flush port 132 can be formed integrally, with plunger 116 being slidably disposed in a proximal end of the singularly formed housing.

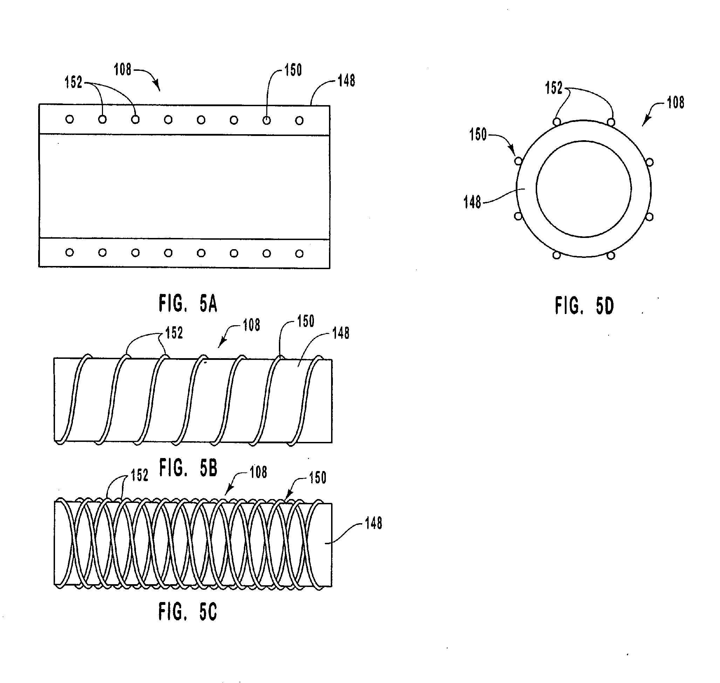

With reference now to Figures 5A through 5D, aspects of capture sleeve 108 will now be discussed in more detail. Capture sleeve 108 can have various characteristics that allow it to effectively capture the filter. The capture sleeve 108 can include one or more of the following: (1) have a small enough diameter to pass through a vascular vessel, particularly, to pass through a stent or other obstructions in the vascular vessel; (2) be sufficiently rigid to be displaced proximally and distally over distal tip 130 by actuation assembly 110; (3) be sufficiently elastic enough to capture components of the filter such as struts; (4) be sufficiently rigid enough to prevent buckling or crumpling when brought in contact with filter components; (5) be sufficiently strong enough to retain the filter therein once the filter has been surrounded or at least partially received by the capture sleeve; and (6) be sufficiently elastic enough to prevent embolic material from squeezing out or "tooth-pasting" from the interior thereof once the filter is captured.

Thus, there is tension between the ability of the capture sleeve 108 to be able to extend and retract in the radial direction while maintaining strength in the axial direction. Filter mechanisms for deploying filter can vary. In some embodiments, the filter mechanism includes struts biased outwardly to release filter. In these embodiments, capture sleeve 108 can be stiff enough to be pushed by deploying member 120 over the struts to close them together. If capture sleeve 108 is too flexible, it will simply crumple against the struts, defeating the intended use of the capture sleeve 108. Capture sleeve 108 can be flexible enough to surround and capture the filter deployment mechanism, but stiff or rigid enough not to kink, wrinkle, or collapse when it exerts force against the filter mechanism. Furthermore, capture sleeve 108 can be strong enough to hold the filter mechanism therein while at the same time, preventing loss of embolic debris from capture sleeve 108.

As discussed above, capture sleeve 108 is slidably disposed or slidably engaged with distal tip 130. Deploying member 120 is rigidly connected to capture sleeve 108 to displace capture sleeve 108 in the proximal and distal direction. During placement of capture catheter 100, capture sleeve 108 is generally in the fully proximal position. That is, the distal end of capture sleeve 108 is disposed over distal tip 130. During deployment, capture sleeve 108 is displaced distally by deploying member 120 in the distal direction to capture or engage the filter media or embolic protection device (not shown). That is, the filter is drawn into the bore of the capture sleeve 108.

As shown in Figure 5A, capture sleeve 108 can include a restraining member 148 cooperating with a reinforcement mechanism or member 150. The restraining member 148 is, in one embodiment, a thin-walled sleeve: Restraining member 148 can be any configuration which allows capture sleeve 108 to at least partially surround the filter and retain the filter therein. The restraining member 148 can be thin-walled to maintain a low profile as it is being positioned in the vessel.

Restraining member 148 can be constructed of plastic, a polymer, or composite material, or a metallic mesh which provides elasticity and resiliency. In another embodiment, restraining member 148 is constructed from a composite material, such as a polymer, stainless steel braid, or other material which provides sufficient flexibility and rigidity and elasticity to perform the desired functions for capture sleeve 108. The length of capture sleeve 108 is such that it can be used to capture filters of various lengths and sizes. Restraining member 148 can also have radiopaque or fluoroscopic markings on the outer surface thereof to assist an operator in placing the capture catheter in the correct location.

Reinforcement mechanism 150 provides radial and axial strength to restraining member 148. As such, capture sleeve 108 is able to perform the above-identified functions. In one embodiment, illustrated in Figure 5A, reinforcement mechanism 150 includes one or more wires 152 embedded in restraining member 148. The one or more wires 152 could be braided within the wall of restraining member 148. The one or more wires 152 could be constructed of plastic, fiber, polymers, steel, alloys, other metals, synthetics, composites, and the like. Wires 152 can be embedded in restraining member 148 by a molding or extrusion process.

In another embodiment, illustrated in Figure 5B, the one or more wires 152 can surround restraining member 148 and be coiled in a circular fashion, but located on the outside of restraining member 148. That is, as depicted in Figure 5D, wires 152 can be disposed on the outside or the inside of restraining member 148 instead of being embedded therein. Wires 152 can be bonded to restraining member 148 by manners or techniques disclosed herein or otherwise known in the art.

In still another embodiment, illustrated in Figure 5C, wires 152 could be configured to have interconnecting joints forming a mesh. This mesh could be embedded or disposed on the inside or outside of restraining member 148.

The reinforcement mechanism 150 allows capture sleeve 108 to be flexible, but reinforces the radial and axial stability of capture sleeve 108 to prevent crushing in the radial or axial direction. In addition, reinforcement mechanism 150 provides axial stability to prevent capture sleeve 108 from collapsing or buckling in the axial direction as it is displaced by actuation assembly 110. In addition, the radial strength provided by reinforcement mechanism 150 prevents the filter from causing the restraining member 148 to bulge outward after capture of the filter so that it does not block the vasculature as it is drawn out, which would cause undue trauma.

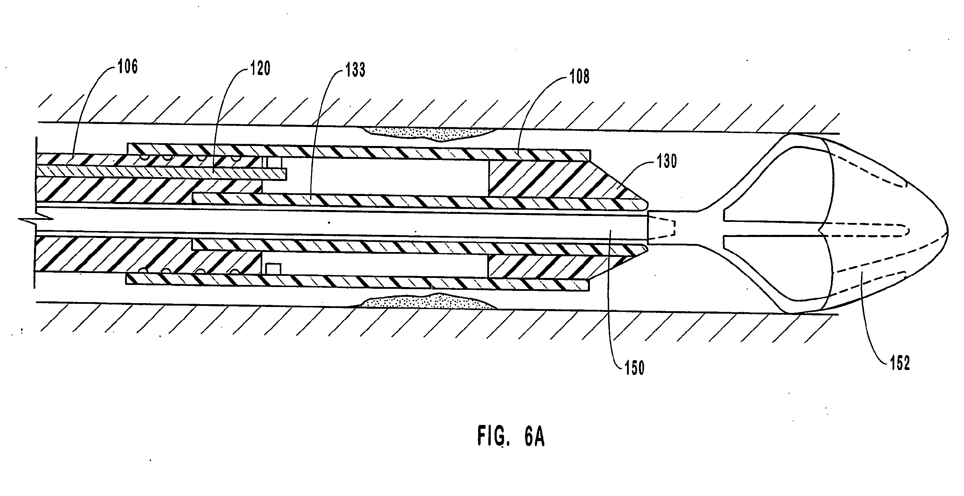

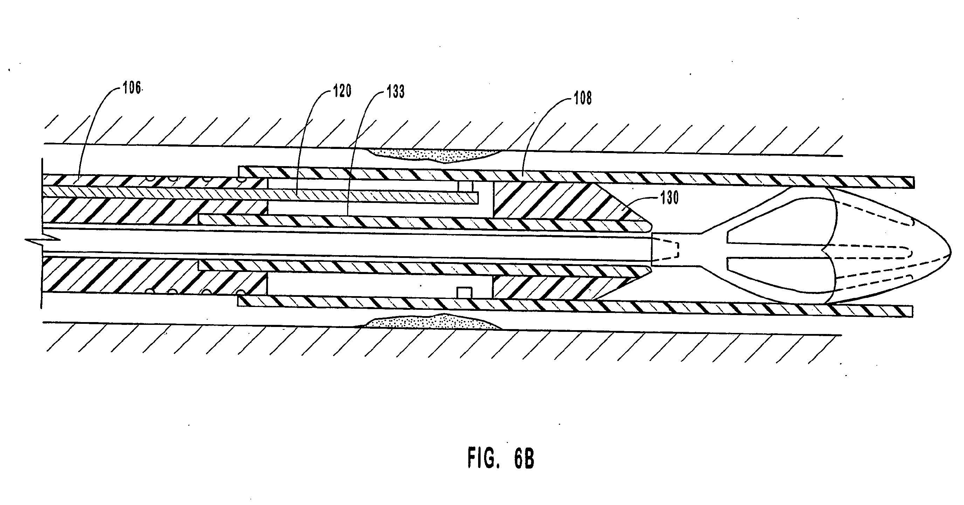

With reference to Figure 6A and 6B, the operation of capture catheter 100 will now be discussed. The operation of capture catheter 100 generally involves multiple phases. In the preparation phase, the air is evacuated in actuation lumen 124 via flush port 132. The actuation assembly 110 is locked or otherwise placed in a "fully loaded" position. That is, plunger 116 is disposed proximally in barrel 112, placing deploying member 120 in a proximal position. This places capture sleeve 108 in a proximal-most position.

Having actuation assembly 110 in a fully loaded position, capture catheter 100 is then inserted into the vascular system. This involves moving the proximal end of a guide wire 150 into guide wire lumen 126. Distal tip 130 then follows guide wire 150 into the vascular system. Distal tip 130 provides for the non-traumatic entrance of capture catheter 100 into the vascular system. The guide wire 150 leads the capture catheter 100 as it is maneuvered and navigated through the blood vessels. As discussed above, guide wire 150 can exit near the distal end of capture catheter 100 in a "rapid exchange" configuration, or, guide wire 150 can exit near the proximal end of capture catheter 100 in a "full exchange" configuration.

Capture catheter 100 then stops at the predetermined location at which a filter 152 or other embolic protection device is located in the blood vessel. Distal end 104 of capture catheter 100 is placed proximally of filter 152. The operator can rely on the tactile feel of capture catheter 100 to maneuver to the desired location. For example, as shown in Figure 6A, the inner diameter of the distal end of distal tip 130 is smaller than the diameter of the proximal end of filter 152. Thus, when the distal end of the capture catheter 100 reaches the filter 152, the distal end of distal tip 130 abuts against the proximal end of the filter. In addition, the operator can monitor fluoroscopic markings on distal end 104 of capture catheter 100 to know when they have reached the appropriate location. Placing capture catheter 100 in the desired location generally requires both hands of the operator.

However, once capture catheter 100 is placed in the desired location, capture sleeve 108 can be deployed using a single hand. Deployment involves pressing head 118 (Figure 1) of plunger 116 (Figure 1), or plunger 116a (Figure 4), to displace deploying member 120 in a distal direction. The movement of deploying member 120 is translated to capture sleeve 108 which is also displaced in a distal direction. As shown in Figure 6B, capture sleeve 108 slides over distal tip 130 to capture filter 152 having the embolic debris contained therein. The capture sleeve 108 causes the struts of filter 152 to bend inwardly so that eventually, at least a portion of the filter is surrounded by the capture sleeve. Because capture catheter 100 contains a distal mechanical stop, the operator is ensured that capture sleeve 108 will not be overextended in the blood vessel. In addition, the operator can rely on markers 140 (Figure 1) located on the outside of barrel 112 to know when capture sleeve 108 has been fully deployed. Once the operator has deployed capture sleeve 108, capture catheter 100 can be locked in the deployed position. The operator can then remove capture catheter 100 from the system, including guide wire 150 and filter 152.

Plunger 116, or plunger 116a, and barrel 112 allow capture sleeve 108 to be advanced variable distances. Thus, depending on the size of the filter 152, capture sleeve 108 does not have to be fully advanced in order to successfully capture the filter. The capture sleeve 108 can be sized such that it can be used to capture filters of various lengths and sizes.

Deployment of capture sleeve 108 only requires one hand, freeing the other hand for other procedures steps. Thus, the operator is not distracted with worrying about losing or moving the guide wire. In existing capture catheters, the operator would feel tactile movement from friction in the vessel or friction over the guide wire. Feeling these friction components would sometimes result in the operator inaccurately placing and/or deploying the capture mechanism. The present capture catheter 100 eliminates at least one source of friction by providing an actuation assembly which is operated axially. In addition, because the actuation assembly is operated at the proximal end of the capture catheter, it reduces the friction components commonly caused by actuation mechanisms at the distal end of the capture catheter. Thus, the operator is not distracted or confused by movements conveyed through the capture catheter, allowing more control over the deployment mechanism.

Furthermore, the mechanical stops provided in the capture catheter prevent the operator from overreaching the filter or overextending the capture sleeve. The mechanical stops prevent the capture sleeve from being extended too far in both the proximal and/or distal directions. For example, if the operator wishes to readjust the positioning of the capture sleeve, without a mechanical stop in the proximal direction, there would be a potential for the capture sleeve to become stuck behind the distal tip 130 since the capture sleeve is made of a resilient material.

Barrel 112 and plunger 116 or 116a are configured to displace deploying member 120 in a linear, axial direction. Barrel 112 and plunger 116 or 116a are particularly convenient because an operator can hold barrel 112 with one hand and actuate plunger 116 or 116a with the thumb. However, barrel 112 and plunger 116 or 116a can be substituted by any mechanism providing axial, sliding movement such that deploying member 120 can be displaced to, in turn, displace capture sleeve 108, as described further below. However, in other embodiments, deploying member 120 can be displaced by rotating plunger 116 or 116a within threads located in barrel 112. This would allow deploying member 120 to be axially displaced and also provide for a more incremental marking system.

In each embodiment of the invention, it is contemplated that the components of capture catheter 100 will have a hydrophilic coating or other coating which prevents components of capture catheter 100 from sticking to the walls of the blood vessel. Suitable coatings have a low friction property such as, but not limited to, polytetrafluoroethylene (PTFE), heparinized coating, or a silicone based coating(s). In addition, it is contemplated that components intended to enter the body will have a thromboresistent coating to prevent blood clots. Alternatively, some portions of capture catheter 100 can be coated while other portions remain uncoated. It will be understood that capture catheter 100 can include one or more coatings.

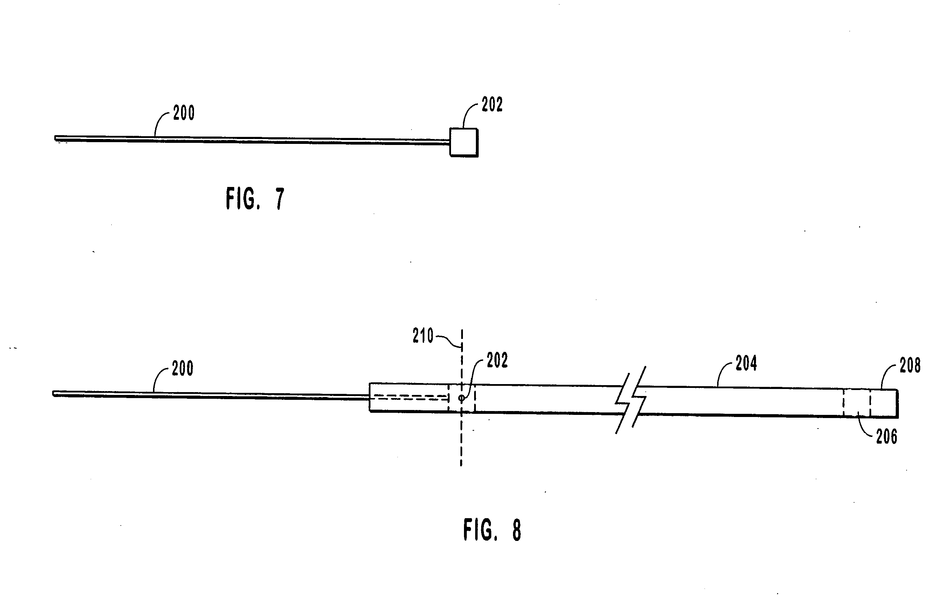

To aid with explaining another embodiment of the present invention, Figures 7-12 illustrate portions of the capture catheter in separated format. Although reference is made to the embodiments illustrated in Figures 7-12, one skilled in the art will appreciate the applicability of the discussion of those embodiments illustrated in Figures 1-6 to the embodiments depicted in Figures 7-12, and vice versa. Discussion will be made with respect to the distal end of a capture catheter 198, while it is understood the proximal end discussed with respect to other embodiments of the present invention are applicable to the discussion of capture catheter 198. Similarly, it will be understood that activation assembly 110 can be used, where applicable, with capture catheter 198.

With reference to Figure 7, illustrated is deploying member 200 coupled or connected to connector member 202. As discussed above, deploying member 200 is a solid wire constructed of stainless steel, shape memory metal, alloy, or a stiff polymer, or synthetic material, like PEEK which will support a compressive load when radially constrained. The deploying member 200 can connect at its proximal end to activation assembly 110 (Figure 1). The connector member 202, coupled to the distal end of deploying member 200, can be a tubular member and can be a length of tubing to which deploying member 200 is attached.

In the illustrated configuration, deploying member 200 can be attached to connector member 202. By so doing, the inner diameter of connector member 202 is preserved. Attaching deploying member 200 to connector member 202 can be achieved by soldering or welding in the case of metallic parts, or by the use of adhesives. If these components are made from polymers, the connection can be achieved by adhesives or solvent bonding. In another configuration, deploying member 200 can be directly attached to a distal or proximal edge of connector member 202. In other configurations, to limit the amount of the inner lumen of connector member 202 that is utilized to attach deploying member 200, a distal end of deploying member 200 can be coined flat or ground flat. Further, connector member 202 can include a recess that receives the distal end of deploying member 200.

Figure 8 shows deploying member 200 and connector member 202 coupled to a capture sleeve 204. In this embodiment, capture sleeve 204 can be generally longer than the one shown in Figure 1. The length of capture sleeve 204 can be from about twenty cm to about fifty cm, although lengths greater and lesser than such lengths are also applicable. The particular length of capture sleeve 204 can be driven by the application that sleeve 202 will be used in, with consideration to keep the proximal end of capture sleeve 204 within a guiding catheter or sheath (not shown) during an interventional procedure. The attachment of capture sleeve 204 with deploying member 200 and connector member 202 can be accomplished by inserting deploying member 200 into an inner diameter of capture sleeve 204.

Generally, the point of connection between connector member 202 and capture sleeve 204 can occur at a position more proximal than the connection point of Figure 1. Once positioned correctly in the length of capture sleeve 204, its position can be secured by an interference fit with the inner diameter of capture sleeve 204, by adhesive, or by some other technique described herein or known to one skilled in the art to connect one member to another member. Having a longer capture sleeve 204 with the connector member 202 coupled at the proximal end of the capture sleeve helps shorten the length of deploying member 200 so that the deploying member is less likely to buckle during deployment. Furthermore, the follower sleeve 142 (Figure 4) can be eliminated if desired.

The capture sleeve 204, in this illustrative configuration, can be a polymer extrusion or co-extrusion that can also be supported by a braid of polymer fibers or metallic wire. The support may also be provided by other synthetic or composite material. The diameter of capture sleeve 204 can be continuous along its length, or transition to a smaller diameter through the center section of its length. The inner diameter can also have lubricious properties which can be accomplished by apply lining it with PTFE or by the use of lubricious coatings which can be sprayed on or applied through a dipping process.

A distal end of capture sleeve 204 contains near its tip a marker 206 which is radiopaque. This marker can be a short length of radiopaque tubing which is embedded in the polymer sleeve material, or a section of polymer which has been filled with a radiopaque material. The very distal tip 208 of capture sleeve 204 can be of a different polymer which is softer than the main section of capture sleeve 204 or that is thinned to provide a softer tip or in the case of a capture sleeve that contains a braid for support, the braid material is removed from this section of the tube. Alternatively, the entire tip section, including radiopaque marker 206 and the very distal tip 208, can be made entirely from a filled, radiopaque polymer material.

To aid in the positioning of deploying member 200 in capture sleeve 204, capture sleeve 204 can be assembled from two or more separate pieces of tubing that are joined together. For instance, as illustrated in Figure 8, two separate piece of tubing are joined together at plane 210, and sleeved over deploying member 200. These separate parts can be attached there by way of an interference fit with the outer diameter of deploying member 200, adhesives which attach it to connector member 202, or by thermal or solvent bonding the separate sections together while attaching to or embedding connector member 202 in the wall.

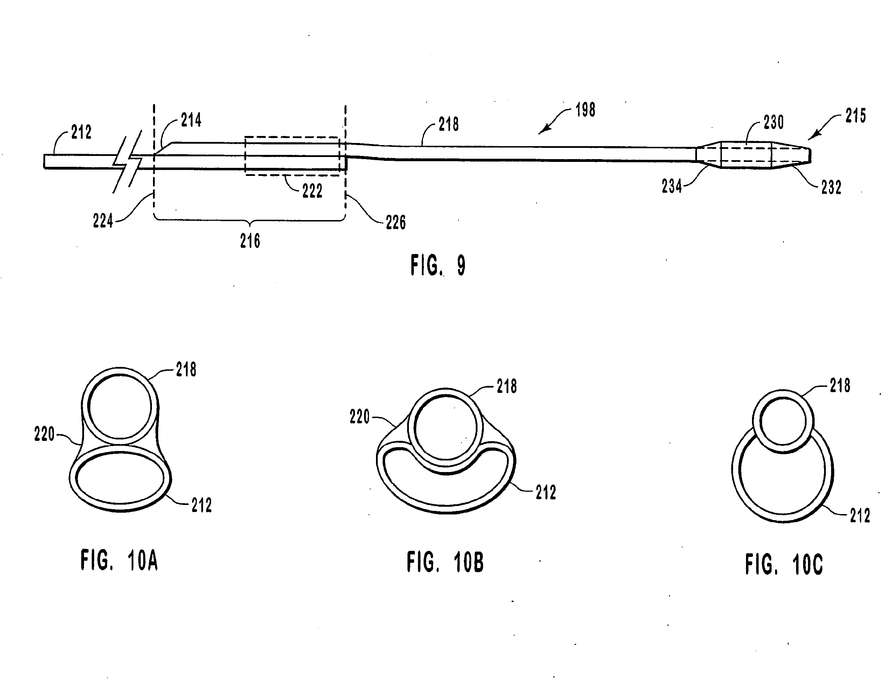

Figure 9 shows a partially assembled capture catheter 198. As illustrated, tubular member 212, such as the catheter shaft of capture catheter 198, is attached to a distal member 218. The catheter shaft or tubular member 212 provides a lumen for deploying member 200, and distal member 218 provides a lumen for a guidewire over which capture catheter 198 can be exchanged. The guidewire can enter distal member 218 at a proximal end 214 and exit from a distal end 215. The distal member 218, therefore, functions in a similar manner to spacer member 133 of Figure 1. Generally, tubular member 212 can be formed from a stainless steel hypo-tube whose outer diameter has been coated to provide a lubricious surface, or a rigid polymer tube. The distal member 218 can be formed from a polymer material such as polyurethane or polyimide.

As illustrated in Figure 9, tubular member 212 and distal member 218 at least partially overlap at a section identified by reference numeral 216. This overlap 216 can be longer than the telescoping length of capture catheter 198. To reduce the outside diameter or cross-sectional profile of capture catheter 198 at overlap 216, flattening of tubular member 212 or distal member 218 can occur, as illustrated in Figure 10A. Flattening of tubular member 212 also aids with attaching tubular member 212 and distal member 218. For instance, overlap 216 or joint can be achieved by the use of adhesives (e.g., UV cured adhesive) between the outer surfaces of tubular member 212 and distal member 218, shown as fillets 220 in Figure 10A. By flattening a portion of tubular member 212 a greater bonding surface area is provided for the adhesive. In addition to or replacing adhesives to connect tubular member 212 and distal member 218, a shrink-type sleeve can surround tubular member 212 and distal member 218 at overlap 216. This heat shrink tubing is illustrated by dotted lines in Figure 9 and identified by reference numeral 222. The shrink tubing 222 can remain at the overlap during use of capture catheter 198 or until curing of the adhesive. Optionally, adhesive fillets 220 in Figures 10A and 10B can also include additional polymer material which is thermal formed, melting onto or into the surfaces of tubular member 212 and distal member 218, thereby joining or fusing tubular member 212 and distal member 218 together.

Various other manners are known to reduce the outside diameter or cross-sectional profile at overlap 216. In another configuration, as illustrated in Figure 10B, tubular member 212 can include a crease or groove that receives a portion of distal member 218. The crease or groove both provides a greater surface area for attaching distal member 218 to tubular member 212 and aids with reducing the overall cross-sectional profile of capture catheter 198. It will be understood that distal member 218 can include the crease or groove and receive a portion of tubular member 212. In still another configuration, as illustrated in Figure 10C, a portion of tubular member 212 can be removed to accommodate distal member 218, or vice versa; thus reducing the outside diameter or cross-sectional profile of overlap 216.

In still another configuration, overlap 216 can also be constructed from a separate, dual lumen, polymer tube that may be bounded by planes 224 and 226. This would allow one lumen of the dual lumen polymer tube to connect to tubular member or catheter shaft 212, while the other lumen would connect to distal member 218. These connections could be adhesive joints, thermal joints, or solvent bonds between the two materials, one of which possibly being polymer.

With continued reference to Figure 9, distal member 218 has at distal end 215 a tip 230 which is adapted to fit closely within an inner diameter of the distal end of capture sleeve 204. Tip 230 serves a similar function as distal tip 130 (Figure 2). The very distal portion of tip 230 has a tapered region 232 extending from a small diameter at the very distal tip to a larger diameter intermediate of tip 230. The proximal end of tip 230 can also have a tapered region 234. The tip 230 can be a separate part from distal member 218 and attached by adhesive, thermal or solvent bonding, or it can be an integral part of distal member 218 and be thermal formed in place on distal member 218. The tip 230, in one configuration, can be formed from Pebax resin which can be formed using an overmolding or insert molding process. In addition, a radiopaque band or marker can be placed on the element 230.

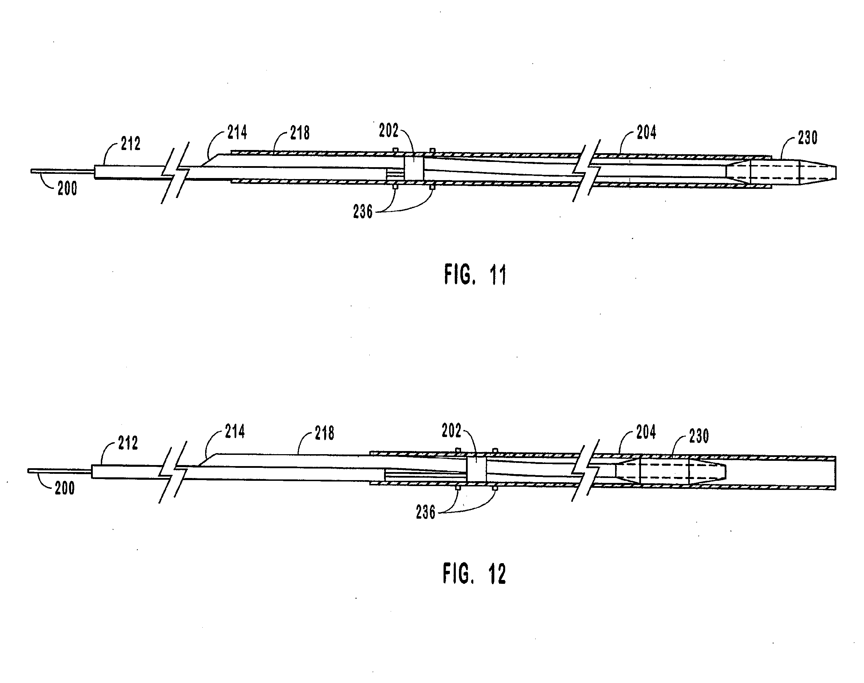

Figure 11 shows the assembled distal end of capture catheter 198. As such, deploying member 200 is inserted into the distal end of tubular member 212, pushed proximally through tubular member 212 and exits its proximal end. It is inserted until the proximal end of capture sleeve 204 encloses overlap or joint 216 and deploying member 200 abuts against the distal end of tubular member 212. The lengths of capture sleeve 204 and distal member 218 are sized so that in the final assembly the very distal end of capture sleeve 204 may be just proximal to the proximal end of tapered region 232 of distal member 218 to provide a continuous, smooth transition from the small guidewire lumen of distal member 218 to the larger, outer diameter of capture sleeve 204.

Figure 12 illustrates capture catheter 198 where capture sleeve 204 is disposed on the tubular member 212 and distal member 218 and is extended over a portion of tip 230. This is achieved as activation assembly 110 (Figures 1, 2, and 4) which moves in a proximal direction causing a resultant displacement of deploying member 200. Since tubular member 212 is fixed relative to deploying member 200, movement of deploying member 200 moves capture sleeve 204 in the distal direction. A pair of reinforcing rings 236 is connected to the outside of capture sleeve 204. One reinforcing ring 236 is placed proximal of connector member 202 and the other is placed distally thereof. These rings 236 can be fabricated from metals, alloys, polymers, composites, synthetics, or other materials capable of forming rings that function to prevent movement of connector member 202 relative to capture sleeve 204.

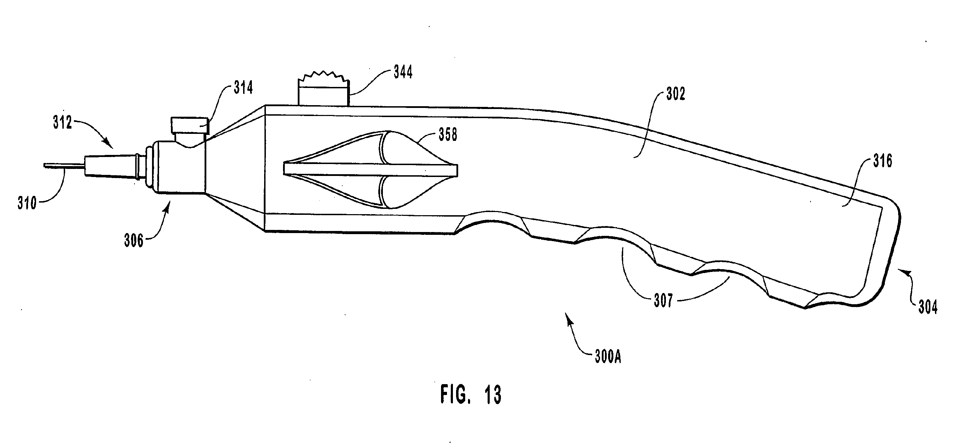

Figures 13 through 17 illustrate other embodiments of actuation assembly, indicated herein as actuation assemblies 300A through 300C that can be used in conjunction with the capture catheter assemblies of the present invention. As shown in Figure 13, actuation assembly 300A includes a housing 302 having a proximal end 304 and a distal end 306. The housing 302 has an ergonomic configuration to accommodate a user's hand. Grips 307 are formed on the outside surface of housing 302 to cooperate with one or more fingers of a user's hand. In addition, the ergonomic configuration of housing 302 allows for the operator to activate a button 344 with one thumb, making operating of the capture catheter simple and effective. In other configuration, the housing may accommodate manipulation of button 34 by one or more fingers rather than the thumb.

Disposed at distal end 306 of housing 302 are a connector hub 312 and a flush port 314. The connector hub 312 provides structure for attaching tubular member 106 or 212. For instance, connector hub 312 can includes threads that engage within complementary threads formed in tubular member 106 or 212. In another configuration, connector hub 312 slip fits, friction fits, or interference fits with tubular member 106 or 212. In still another configuration, connector hub 312 bonds or otherwise attaches to tubular member 106 or 212.

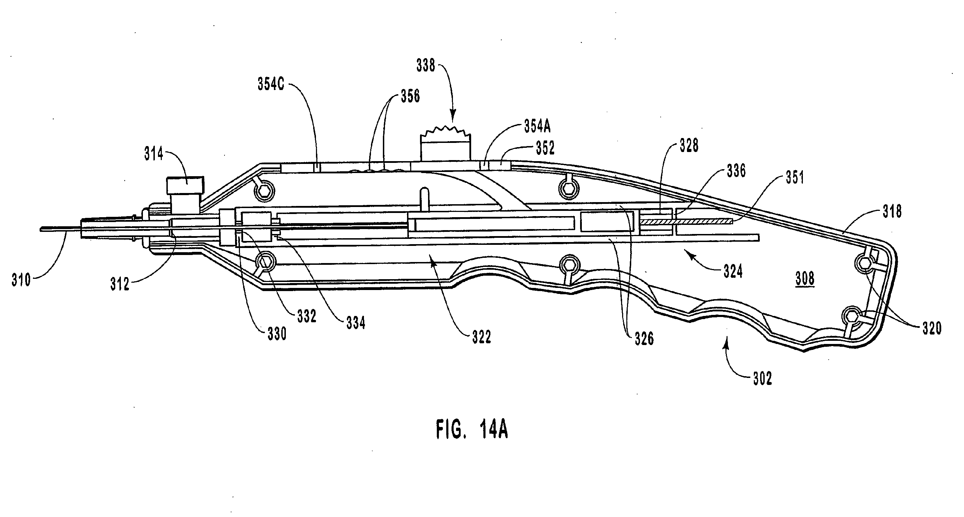

Housing 302 can generally be formed from two mating portions. For example, Figure 13 could represent one portion 316 of housing 302, while Figure 14A could represent another portion 318 of housing 302. The portions 316, 318 can be connected together, for example, via pins (not shown) formed on portion 316 and receiving structures with holes 320 formed on portion 318. Portions 316, 318 can then be bolted, welded, riveted, or connected by a suitable adhesive.

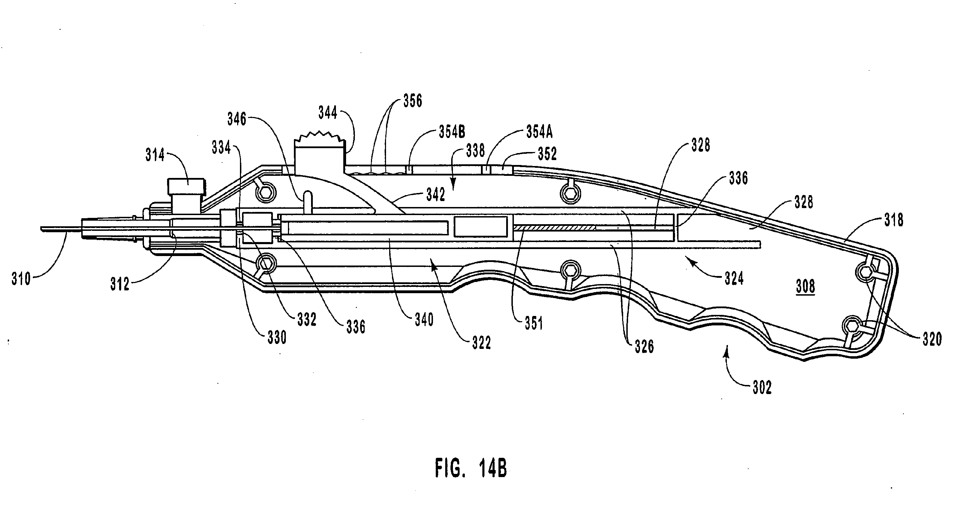

As shown in Figure 14A, housing 302 has a sliding assembly 322. Sliding assembly 322 can include a track 324 which cooperates with an actuating member 338. The track 324 allows actuating member 338 to be selectively positioned between a proximal position (Figure 14A) and a distal position (Figure 14B). The actuating member 338 connects to deployment member 310, such that movement of actuating member 338 simultaneously displaces deployment member 310 between a proximal position and a distal position.

In the illustrated configuration, track 324 includes two outer rails 326 and a support rail 328. These rails 326 and 328 both guide and support actuating member 338 as it moves from a proximal position to a distal position. Rails 326 and 328 terminate in stops 334 and 336 that limit movement of actuating member 338. These stops 334 and 336 can be located at any location between a proximal end and a distal end of track 324. Generally, track 324 can be formed integrally with housing 302 where the housing and track are constructed from plastic, e.g., by injection molding. In another embodiment, housing 302 and track 324 can be integrally formed from metal. In yet another embodiment, track 324 can be mounted separately into chamber 308 of housing 302 after formation thereof. Further, track 324 can include one or more outer rails and one or more support rails. It will be understood that rails 326 and 328 can both support and guide a portion of actuating member 338.

As shown in Figure 14A, disposed at the distal end of outer rails 326 is a wall 330. Wall 330 can connect to the outer rails 326, be integrally formed with outer rails 326 and/or support rail 328, or be a separate element that mounts to housing 302. Wall 330 includes an aperture 332 which is configured to slidably receive deployment member 310. A seal 334 can be placed in aperture 332 to seal aperture 332 from connector hub 312 so that flushing fluid does not enter chamber 308.

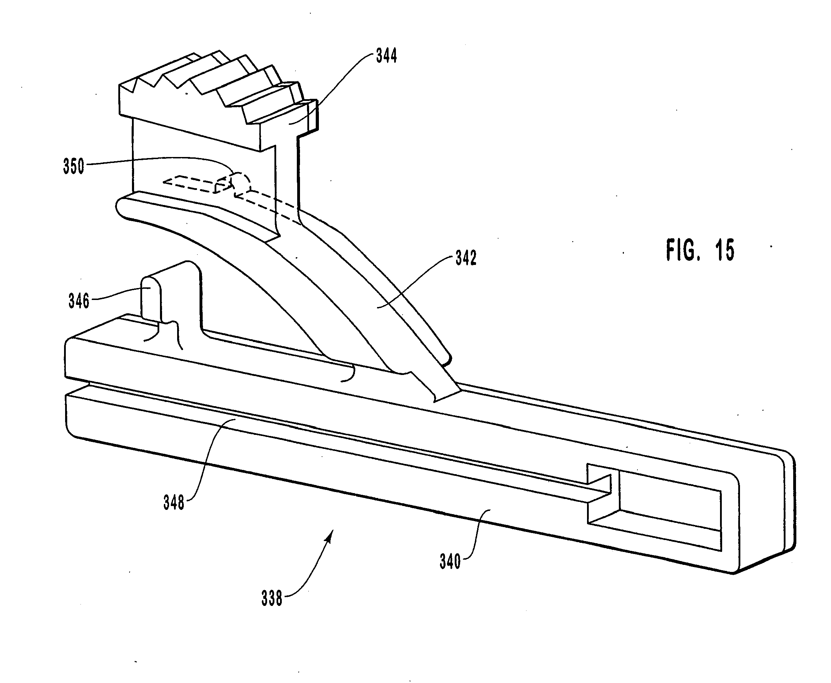

With further attention to Figure 14A, sliding assembly 322 also includes an actuating member 338 slidably disposed on track 324. Actuating member 338 connects to and receives deployment member 310. With reference to Figure 15, actuating member 338 includes a body 340 that is configured to mount within track 324. Both the body 340 and track 324 have complementary configurations to enable slidably movement of body 340 along track 324. A flexible arm 342 extends from body 340 and terminates with a button or lever 344. Additionally, a compression limiter 346 extends from body 340. Compression limiter 346 is sized so that when an operator depresses flexible arm 342, resulting in flexible arm 342 moving toward body 340, arm 342 will come in contact with compression limiter 346. Thus, compression limiter 346 limits movement of arm 342 toward body 340 during use of actuation assembly 300.

With continued reference to Figure 15, actuating member 338 also include a channel 348 to receive deployment member 310 and optionally a portion of support rail 328. In addition, a protruding portion 350 is also formed on arm 342. Protruding portion 350 cooperates with housing 302 to form incremental mechanical stops, which will be described in more detail below.

As discussed above, deployment member 310 can be coupled to actuating member 338. As shown in Figure 14A, a reinforcing tube 351 is provided to receive the proximal end of deployment member 310. Deployment member 310 can be connected to reinforcing tube 351 through adhesives, composite adhesives, complementary threads, slip-fit, friction fit, or interference fit bonds, or various other techniques, such as but not limited to, welding, soldering, thermal bonding, chemical bonding, or other techniques that facilitate securely or releasably disposing a tube within a channel. Central track 328 and, consequently, channel 348 can align with aperture 332 of wall 330. Reinforcing tube 351 can be placed in channel 348 such that deployment member 310 aligns with aperture 332 of wall 330. The channel 348 can be constructed deep enough to accommodate both reinforcing tube 351 and support rail 328 of track 324. Aperture 332, in turn, aligns with connector hub 312 so that deployment member 310 extends therefrom. In another embodiment, support track 328 and channel 348 can be displaced above or below aperture 332 of wall 330 and reinforcing tube 351 can be connected to actuating member 338 so that it aligns with aperture 332. In another embodiment (not shown), the deployment member can be directly connected to the proximal end of actuating member 338, such as through insert molding, adhesives, complementary threads, slip-fit, friction fit, or interference fit bonds, or various other techniques, such as but not limited to, welding, thermal bonding, chemical bonding, or other techniques that facilitate securely or releasably disposing a tube within a channel.

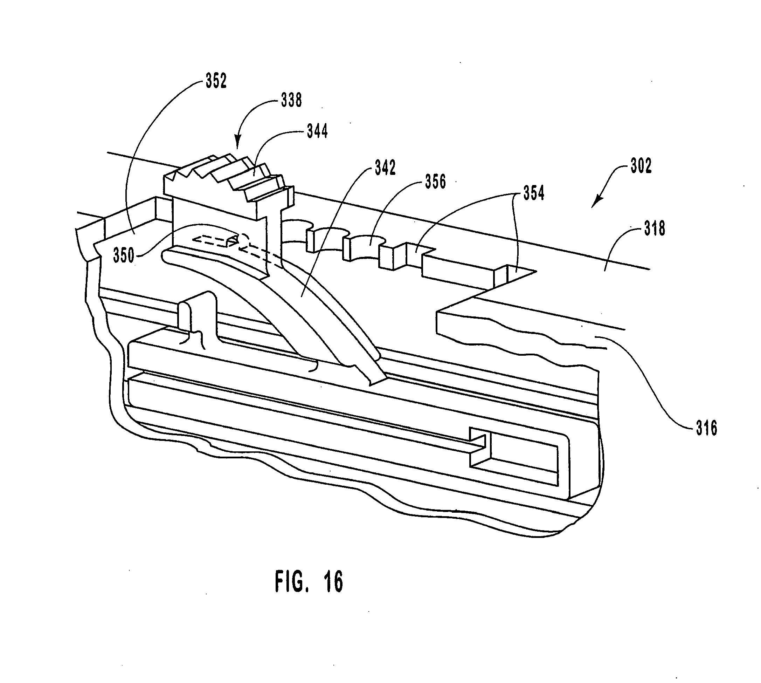

With reference still to Figure 16, a slot 352 can be formed on the surface of housing 302, for example, between the interfaces of portions 316, 318. Arm 342 and button 344 are disposed in slot 352 such that a large enough portion of button 344 extends therethrough so that a person can access and operate the button. One side of slot 352 includes one or more locking grooves or detents 354 within which a portion of arm 342 can be configured to be received. The other side of slot 352 can be substantially linear so that arm 342 of actuating member 338 can slide freely thereagainst.

Between grooves 354, the inside surface of slot 352 can include a plurality of depressions 356. Depressions 356 selectively mate with protrusion portion 350 on actuating member 338 to provide the operator with tactile feel as the actuating member 338 can be selectively positioned between grooves 354. In addition, depression 356 and protrusion portion 350 can operate to provide an incremental locking mechanism as the actuating member 338 can be moved from one groove 354 to another.

As shown in Figures 14A and 14B, actuating member 338 can be slidably disposed on track 324 so that it can be selectively operated in the proximal and/or distal directions. Thus, moving actuating member 338 relative to housing 302 causes deploying member 310 to move relative to the tubular member (not shown). It will be appreciated that such movement also actuates movement of the capture mechanism (not shown) disposed at the distal end of the capture catheter.

The maximum proximal and/or distal positions of actuating member 338 can be determined by mechanical stops. Proximal and distal stops can be provided by stops 334 and 336 in track 324. In addition, locking grooves 354 can form mechanical stops in embodiments where arm 342 of actuating member 338 can be biased to be disposed in locking grooves 354. Finally, depressions 356 and protrusion 350 can form incremental stops. These mechanical stops can be used to reference the operator between starting and stopping points during the capture procedure. Other mechanical stops may be provided as understood by those skilled in the art in view of this disclosure. In addition, as shown in Figure 13, a marker 358 can be placed on the outside surface of housing 302 in order to provide the operator a visual reference when operating the capture catheter. It will be appreciated that the mechanical stops can also act as a locking mechanism.

In one embodiment; grooves 354 or depressions 356 of slot 352 enable actuating member 338 to be locked into at least three positions, noted in Figures 14A and 14B as storage position 354A, proximal position 354B and distal position 354C. When the actuation assembly 300 can be being stored or shipped, the actuating member 338 can be positioned in a storage position by placing arm 342 into storage position 354A. During operation of the capture catheter with which actuation assembly 300 can be associated, actuating member 338 can be placed in the proximal position 354B (see Figure 14A). During deployment of the capture mechanism, the actuating member 338 can be moved from the proximal position 354B to the distal position 354C to enable the deployment mechanism to be activated (Figure 14B).

In one configuration, arm 342 can be biased to be disposed in one of the grooves 354 or depressions 356 in order to lock actuating member 338 in place. When an operator desires to reposition actuating member 338, the operator can push the button 344 sideways to release the arm 342 from one groove 354 or depression 356 and then will be able to freely slide the actuating member 338 along slot 352 by allowing the arm to slide against the side of the slot opposite that which contains the grooves. Upon releasing the force applied to arm 342 following moving arm 342 the desired longitudinal distance, protrusion 350 reengages one of grooves 354 or depressions 356.

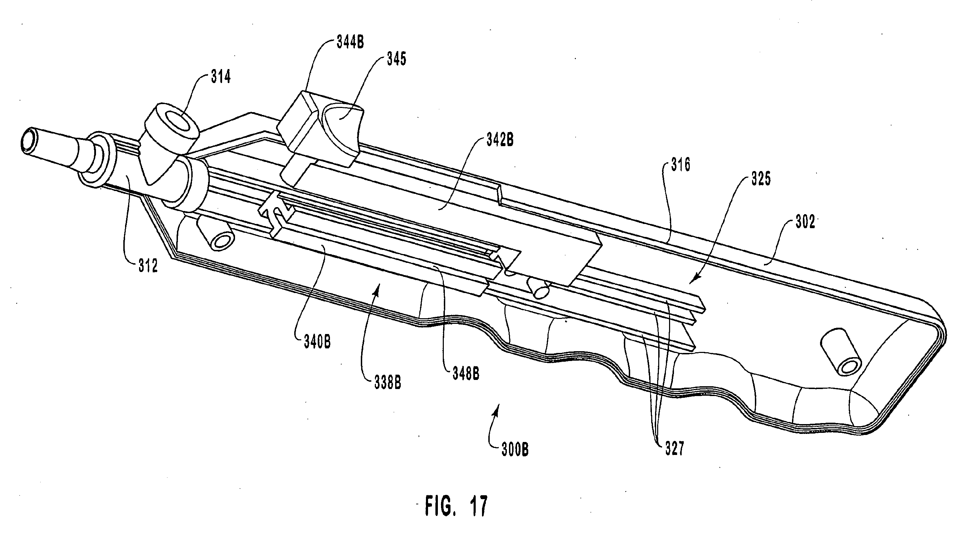

With reference to Figure 17, another embodiment of actuation assembly 300B is shown. Actuation assembly 300B can be substantially similar to actuation assembly 300A described above and so like elements will be referred to with like reference numerals. Figure 17 shows a portion 316 of housing 302 having actuating member 338B disposed therein. Housing 302 can be a different shape form the housing in Figure 13. However, portion 316 can have features complementary to those of Figure 13. Figure 17 illustrates that portion 316 can include a track 325 that can be complementary to track 324. That is, track 325 provides one or more rails 327 shaped so that actuating member 338B can slide thereagainst. Tracks 324 and 325 cooperate to hold actuating member 338B securely therebetween when portions 316, 318 of housing 302 are coupled together. In addition, Figure 17 illustrates that the portion of slot 352 formed on portions 316 and 318 of housing 302 can be substantially rectilinear without any grooves or depressions formed therein. This allows actuating member 338B to slide smoothly thereagainst and provides the operator with infinitesimal position between the proximal and distal ends of slot 352. When portions 316, 318 of housing 302 are coupled together, slot 352 thus has two smooth sides.

In the embodiment of Figure 17, actuating member 338B includes a body 340B, an arm 342B which can be connected to a proximal end of body 340B and can be substantially parallel to body 340B, and a button 344B disposed at the distal end of arm 342B. The button 344B can include curved portion 345 that accommodates a thumb of an operator. A sliding channel 348B can be also formed in body 340B. Similar to the embodiment described above, arm 342B can be spring-biased so that it rests in the grooves 354 formed in slot 352.

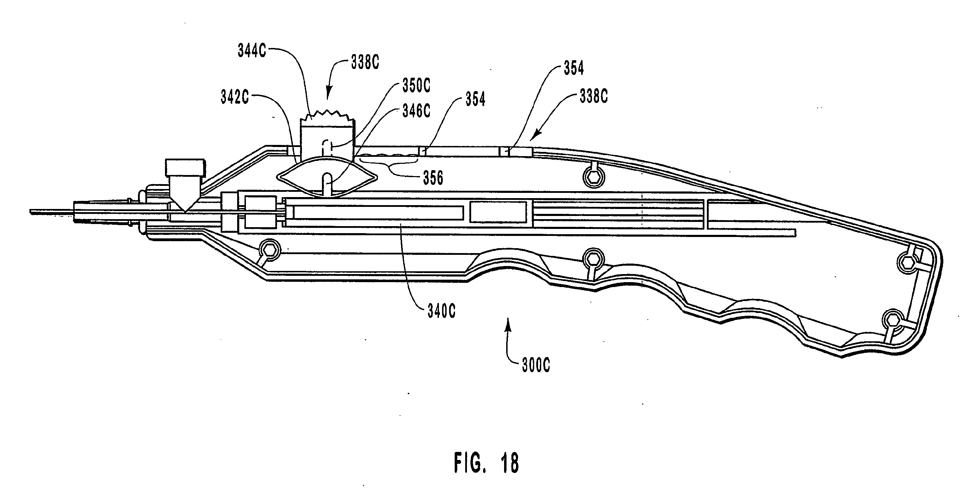

Referring now to Figure 18, another embodiment of an actuating member 338C is illustrated. In this embodiment, actuating member 338C has a body 340C with a leaf spring 342C. A button 344C can be disposed on spring 342C. Button 344C can also include a protruding portion 350C extending outward and upward from the button so that it can be selectively disposed in locking grooves 354 or depression 356 in slot 352. Button 344C can be operable to release actuating member 338C from locking grooves 354 in slot 352 by applying force to spring 342C which, upon being depressed, removes protruding portion 350C from one of locking groove 354 or depressions 356. The actuating member 338C can be slid along track 324 until protruding portion 350C comes into contact with the same or another locking groove 354 or depression 356. This embodiment may be desired because the operator simply has to press downward and then longitudinally, rather than outward and longitudinally.

Embodiments of the present invention provide a capture catheter that can be configured to capture an embolic protection device that collects embolic material after it has been deployed during a surgical procedure. Positioning and operating the capture catheters of the present invention are easier to operate, capable of being manipulated using a single hand, while preventing positioning of the capture catheter at a location inapposite to effective retrieval or capturing of a deployed embolic protection device.

The present invention can be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A capture catheter (100, 198) capable of being displaced along a guidewire within a body lumen and to engage with an embolic protection device that collects embolic material, the capture catheter (100, 198) comprising:
an elongate member (106, 212) having a proximal end (102) and an atraumatic distal end (104, 215), the elongate member (106, 212) comprising a first lumen (124) extending from said proximal end (102) toward said distal end (104, 215) thereof and a second lumen (126) extending from said distal end (104, 215) toward said proximal end (102);
a capture sleeve (108, 204) slidably cooperating with said distal end (104, 215) of said elongate member (106, 212); and
an actuating assembly (110, 300, 300A, 300B, 300C) connected to said proximal end (102) of said elongate member (106, 212), said actuating assembly (110, 300, 300A, 300B, 300C) having a deploying member (120, 200, 310) that couples with said capture sleeve (108, 204) and slidably cooperates with said first lumen (124) so that an operator can move said deploying member (120, 200, 310) in a controlled manner using a single hand of the operator;
wherein the atraumatic distal end (104, 215) acts as a transition between an outside diameter of the guide wire and the capture catheter (100, 198) and aids with maneuvering and navigating the capture catheter (100, 198) through the body lumen.

2. The capture catheter (100, 198) as recited in claim 1, wherein said actuating assembly comprises:
a barrel (112) connected to said proximal end (102) of said elongate member (106, 212); and
a plunger (116, 116a) slidably disposed in said barrel (112), said plunger (116, 116a) being connected to said deploying member (120, 200, 310).

3. The capture catheter (100, 198) as recited in claim 1, wherein said actuating assembly (110, 300, 300A, 300B, 300C) comprises:
a housing (302); and
a sliding assembly (322) disposed in said housing (302), said sliding assembly (322) being connected to said deployment member (120, 200, 310) such that said deployment member (120, 200, 310) can be moved proximally and distally.

4. The capture catheter (100, 198) as recited in claim 3, wherein said sliding assembly (322) comprises:
a slot (352) formed on a surface of said housing (302);
a track (324) disposed in said housing (302); and
an actuating member (338, 338B, 338C) slidably disposed on said track (324), said deployment member (120, 200, 310) being connected to said actuating member (338, 338B, 338C).

5. The capture catheter (100, 198) as recited in claim 4, wherein said actuating member (338, 338B, 338C) comprising a body (340, 340B, 340C), and a flexible arm (342) depending outwardly from said body (340, 340B, 340C) and at least partially extending through said slot (352) of said housing (302) such that said flexible arm (342) is accessible by an operator to move said actuating member (338, 338B, 338C) along said track (324).

6. The capture catheter (100, 198) as recited in claim 4, wherein said slot (352) includes at least one locking groove (354) formed along a length thereof, wherein said flexible arm (342) can be selectively disposed in said at least one locking groove (354).

7. The capture catheter (100, 198) as recited in claim 4, further comprising a plurality of tactical ribs formed on an inside surface of said housing (302).

8. The capture catheter (100, 198) as recited in claim 4, further comprising a compression limiter (346) formed on said body (340, 340B, 340C) of said actuating member (338, 338B, 338C).

9. The capture catheter (100, 198) as recited in claim 1, wherein said actuating assembly (110, 300, 300A, 300B, 300C) further comprises markings on a housing (302) of said actuating assembly (110, 300, 300A, 300B, 300C), said markings providing indicators as to the deployed and un-deployed state of said capture sleeve (108, 204).

10. The capture catheter (100, 198) as recited in claim 1, wherein said actuating assembly (110, 300, 300A, 300B, 300C) further comprises one or more stops (334, 336) that limit the movement of said deploying member (120, 200, 310) during deployment and retrieval of the embolic protection device.

11. The capture catheter (100, 198) as recited in claim 1, further comprising a secondary sleeve (142) coupled to said capture sleeve (108, 204), said secondary sleeve (142) extending in a proximal direction from a proximal end of said capture sleeve (108, 204).

12. The capture catheter (100, 198) as recited in claim 1, wherein said capture sleeve (108, 204) further comprises:
a tubular sleeve configured to at least partially surround the embolic protection device and to retain the embolic protection device therein without losing a significant amount of embolic debris therefrom; and
a reinforcement mechanism (150) cooperating with said tubular sleeve to provide said tubular sleeve with radial and axial strength to prevent said tubular sleeve from buckling or collapsing against the embolic protection device.

13. The capture catheter (100, 198) as recited in claim 1, wherein said deploying member (120, 200, 310) comprises reinforcement mechanisms at both ends to prevent said deploying member (120, 200, 310) from buckling or collapsing during displacement of said deploying member (120, 200, 310).

14. The capture catheter (100, 198) as recited in claim 1, wherein
the first lumen (124) is configured to receive the guide wire and the second lumen (126) is configured to receive the deploying member (120, 200, 310), said atraumatic distal end (104, 215) being adapted to prevent movement of said elongate member (106, 212) distal to a proximal end of the embolic protection device; and
wherein the capture sleeve (108, 204) slidably cooperates with at least a portion of an exterior surface of said elongate member (106, 212).

15. The capture catheter (100, 198) as recited in claim 14, wherein said actuating assembly comprises:
a barrel (112) connected to said proximal end (102) of said elongate member (106, 212), said barrel (112) having a chamber (115) formed therein; and
a plunger (116, 116a) slidably engaging with at least a portion of said chamber (115), said plunger (116, 116a) being connected to said deploying member (120, 200, 310) such that moving said plunger (116, 116a) moves said deploying member (120, 200, 310).

16. The capture catheter (100, 198) as recited in claim 15, wherein said barrel (112) is integrally formed with said elongate member (106, 212).

17. The capture catheter (100, 198) as recited in claim 15, wherein said plunger (116, 116a) comprises an outer telescoping member (134) and an inner telescoping member (136) slidably disposed about said elongate member (106, 212).

18. The capture catheter (100, 198) as recited in claim 17, wherein said inner telescoping member (136) is bonded around said end of said deploying member (120, 200, 310) to reinforce said deploying member (120, 200, 310).

19. The capture catheter (100, 198) as recited in claim 14, wherein said actuating assembly comprises:
a housing (302);
a slot (352) formed on a surface of the housing (302);
a track (324) disposed in the housing (302); and
an actuating member (338, 338B, 338C) slidably disposed on the track (324), said deployment member (120, 200, 310) being connected to said actuating member (338, 338B, 338C).

20. The capture catheter (100, 198) as recited in claim 19, wherein said actuating member (338, 338B, 338C) comprising a body (340, 340B, 340C), and a flexible arm (342) depending outwardly from said body (340, 340B, 340C) and at least partially extending through said slot (352) of said housing (302) such that said flexible arm (342) is accessible by an operator to move said actuating member (338, 338B, 338C) along said track (324).

21. The capture catheter (100, 198) as recited in claim 14, wherein said capture sleeve comprises:
a tubular sleeve; and
a reinforcement mechanism (150) disposed in cooperation with said tubular sleeve to provide axial and radial strength to said tubular sleeve.

22. The capture catheter (100, 198) as recited in claim 14, wherein said capture sleeve (108, 204) comprises a tubular sleeve.

23. The capture catheter (100, 198) as recited in claim 14, further comprising a transition sleeve disposed over at least a portion of an exterior surface of said elongate member (106, 212) and at least a portion of said exterior surface of said capture sleeve (108, 204).

24. The capture catheter (100, 198) as recited in claim 14, wherein said second lumen (126) terminates proximal to a distal end of said elongate member (106, 212).

25. The capture catheter (100, 198) as recited in claim 14, further comprising a secondary sleeve (142) coupled to said capture sleeve (108, 204), wherein said secondary sleeve (142) extends from said capture sleeve (108, 204) to a location where said second lumen terminates upon said capture sleeve (108, 204) being moved a maximum distance in a distal direction.

26. The capture catheter (100, 198) as recited in claim 24, wherein said secondary sleeve (142) prevents buckling of said deploying member (120, 200, 310) as said capture sleeve (108, 204) is moved in the distal direction.

27. The capture catheter (100, 198) as recited in claim 14, wherein moving said deploying member (120, 200, 310) displaces said capture sleeve (108, 204) relative to said elongate member (106, 212) to engage with or release at least a portion of the embolic protection device.

## Patentansprüche

1. Aufnahmekatheter (100, 198), der entlang eines Führungsdrahtes innerhalb eines Körperlumens bewegbar ist, und mit einer Embolieschutzvorrichtung, die Emboliematerial sammelt, in Eingriff bringbar ist, wobei der Aufnahmekatheter (100, 198) aufweist:
ein längliches Teil (106, 212) mit einem proximalen Ende (102) und einem atraumatischen distalen Ende (104, 215), wobei das längliche Teil (106, 212) aufweist: ein erstes Lumen (124), das sich von dem proximalen Ende (102) in Richtung des distalen Endes (104, 215) davon erstreckt, und ein zweites Lumen (126), das sich von dem distalen Ende (104, 215) in Richtung des proximalen Endes (102) erstreckt;
eine Aufnahmehülse (108, 204), die mit dem distalen Ende (104, 215) des länglichen Teils (106, 212) gleitfähig zusammenwirkt; und
eine Betätigungsanordnung (110, 300, 300A, 300B, 300C) die mit dem proximalen Ende (102) des länglichen Teils (106, 212) verbunden ist, wobei die Betätigungsanordnung (110, 300, 300A, 300B, 300C) ein Entfaltungsteil (120, 200, 310) hat, das mit der Aufnahmehülse (108, 204) gekoppelt ist und mit dem ersten Lumen (124) gleitfähig zusammenwirkt, so dass ein Benutzer das Entfaltungsteil (120, 200, 310) auf eine kontrollierte Art und Weise unter Verwendung einer einzigen Hand des Benutzers bewegen kann;
wobei das atraumatische distale Ende (104, 215) als Übergang zwischen einem Außendurchmesser des Führungsdrahts und des Aufnahmekatheters (100, 198) wirkt und die Manövrierung und Navigierung des Aufnahmekatheters (100, 198) durch das Körperlumen unterstützt.

2. Aufnahmekatheter (100, 198) nach Anspruch 1, wobei die Betätigungsanordnung aufweist:
einen Zylinder bzw. Lauf (112), der mit dem proximalen Ende (102) des länglichen Teils (106, 212) verbunden ist; und
einen Kolben (116, 116a), der in dem Zylinder bzw. Lauf (112) gleitfähig angeordnet ist, wobei der Kolben (116, 116a) mit dem Entfaltungsteil (120, 200, 310) verbunden ist.

3. Aufnahmekatheter (100, 198) nach Anspruch 1, wobei die Betätigungsanordnung (110, 300, 300A, 300B, 300C) aufweist:
ein Gehäuse (302); und
eine Gleitanordnung (322), die in dem Gehäuse (302) angeordnet ist, wobei die Gleitanordnung (322) mit dem Entfaltungsteil (120, 200, 310) verbunden ist, so dass das Entfaltungsteil (120, 200, 310) proximal und distal bewegt werden kann.

4. Aufnahmekatheter (100, 198) nach Anspruch 3, wobei die Gleitanordnung (322) aufweist:
einen Schlitz (352), der auf einer Fläche des Gehäuses (302) ausgebildet ist;
eine Führungsbahn (324), die in dem Gehäuse (302) angeordnet ist; und
ein Betätigungsteil (338, 338B, 338C), das in der Führungsbahn (324) gleitfähig angeordnet ist, wobei das Entfaltungsteil (120, 200, 310) mit dem Betätigungsteil (338, 338B, 338C) verbunden ist.

5. Aufnahmekatheter (100, 198) nach Anspruch 4, wobei das Betätigungsteil (338, 338B, 338C) aufweist: einen Körper (340, 340B, 340C) und einen flexiblen Arm (342), der von dem Körper (340, 340B, 340C) nach außen vorsteht und sich zumindest teilweise durch den Schlitz (352) des Gehäuses (302) erstreckt, so dass der flexible Arm (342) derartig zugänglich ist, dass ein Benutzer das Betätigungsteil (338, 338B, 338C) entlang der Führungsbahn (324) bewegen kann.

6. Aufnahmekatheter (100, 198) nach Anspruch 4, wobei der Schlitz (352) zumindest eine Verriegelungsnut (354) aufweist, die entlang seiner Länge ausgebildet ist, wobei der flexible Arm (342) in der zumindest einen Verriegelungsnut (354) selektiv angeordnet werden kann.

7. Aufnahmekatheter (100, 198) nach Anspruch 4, ferner mit mehreren taktischen Rippen, die an einer Innenfläche des Gehäuses (302) ausgebildet sind.

8. Aufnahmekatheter (100, 198) nach Anspruch 4, ferner mit einem Kompressionbegrenzer (346), der an dem Körper (340, 340B, 340C) des Betätigungselements (338, 338B, 338C) ausgebildet ist.

9. Aufnahmekatheter (100, 198) nach Anspruch 1, wobei die Betätigungsanordnung (110, 300, 300A, 300B, 300C) ferner Markierungen an einem Gehäuse (302) der Betätigungsanordnung (110, 300, 300A, 300B, 300C) aufweist, wobei die Markierungen Indikatoren bezüglich des entfalteten und nichtentfalteten Zustands der Aufnahmehülse (108, 204) bereitstellen.

10. Aufnahmekatheter (100, 198) nach Anspruch 1, wobei die Betätigungsanordnung (110, 300, 300A, 300B, 300C) ferner einen oder mehrere Anschläge (334, 336) aufweist, die die Bewegung des Entfaltungsteils (120, 200, 310) während der Entfaltung und Rückholung der Embolieschutzvorrichtung begrenzen.

11. Aufnahmekatheter (100, 198) nach Anspruch 1, ferner mit einer sekundären Hülse (142), die mit der Aufnahmehülse (108, 204) gekoppelt ist, wobei die sekundäre Hülse (142) sich in einer proximalen Richtung von einem proximalen Ende der Aufnahmehülse (108, 204) erstreckt.

12. Aufnahmekatheter (100, 198) nach Anspruch 1, wobei die Aufnahmehülse (108, 204) ferner aufweist:
eine röhrenförmige Hülse, die entsprechend konfiguriert ist, um die Embolieschutzvorrichtung zumindest teilweise zu umgeben und die Embolieschutzvorrichtung darin zu halten, ohne eine erhebliche Menge an Emboluspartikeln daraus zu verlieren; und
einen Verstärkungsmechanismus (150), der mit der röhrenförmigen Hülse zusammenwirkt, um die röhrenförmige Hülse mit radialen und axialen Kräften zu versehen, um zu verhindern, dass die röhrenförmige Hülse einknickt oder gegen die Embolieschutzvorrichtung kollabiert.

13. Aufnahmekatheter (100, 198) nach Anspruch 1, wobei das Entfaltungsteil (120, 200, 310) Verstärkungsmechanismen an beiden Enden aufweist, um zu verhindern, dass das Entfaltungsteil (120, 200, 310) während der Verschiebung des Entfaltungsteils (120, 200, 310) einknickt oder kollabiert.

14. Aufnahmekatheter (100, 198) nach Anspruch 1, wobei
das ersten Lumen (124) entsprechend konfiguriert ist, um den Führungsdraht aufzunehmen, und das zweite Lumen (126) entsprechend konfiguriert ist, um das Entfaltungsteil (120, 200, 310) aufzunehmen, wobei das atraumatische distale Ende (104, 215) dafür vorgesehen bzw. ausgebildet ist, eine Bewegung des länglichen Teils (106, 212) distal zu einem proximalen Ende der Embolieschutzvorrichtung zu verhindern; und
wobei die Aufnahmehülse (108, 204) mit zumindest einem Abschnitt einer Außenfläche des länglichen Teils (106, 212) gleitfähig zusammenwirkt.

15. Aufnahmekatheter (100, 198) nach Anspruch 14, wobei die Betätigungsanordnung aufweist:
einen Zylinder bzw. Lauf (112), der mit dem proximalen Ende (102) des länglichen Teils (106, 212) verbunden ist, wobei in dem Zylinder bzw. Lauf (112) eine Kammer (115) entsprechend ausgebildet ist; und
einen Kolben (116, 116a), der mit zumindest einem Abschnitt der Kammer (115) gleitfähig in Eingriff ist, wobei der Kolben (116, 116a) mit dem Entfaltungsteil (120, 200, 310) verbunden ist, so dass durch die Bewegung des Kolbens (116, 116a) das Entfaltungsteil (120, 200, 310) bewegt wird.

16. Aufnahmekatheter (100, 198) nach Anspruch 15, wobei der Zylinder bzw. Lauf (112) mit dem länglichen Teil (106, 212) einstückig ausgebildet ist.

17. Aufnahmekatheter (100, 198) nach Anspruch 15, wobei der Kolben (116, 116a) ein äußeres Teleskopteil (134) und ein inneres Teleskopteil (136) aufweist, die um das längliche Teil (106, 212) gleitfähig angeordnet sind.

18. Aufnahmekatheter (100, 198) nach Anspruch 17, wobei das innere Teleskopteil (136) um das Ende des Entfaltungsteils (120, 200, 310) verbunden angeordnet ist, um das Entfaltungsteil (120, 200, 310) zu verstärken.

19. Aufnahmekatheter (100, 198) nach Anspruch 14, wobei die Betätigungsanordnung aufweist:
ein Gehäuse (302),
einen Schlitz (352), der an einer Oberfläche des Gehäuses (302) ausgebildet ist;
eine Führungsbahn (324), die in dem Gehäuse (302) angeordnet ist; und
ein Betätigungsteil (338, 338B, 338C), das in der Führungsbahn (324) gleitfähig angeordnet ist, wobei das Entfaltungsteil (120, 200, 310) mit dem Betätigungsteil (338, 338B, 338C) verbunden ist.

20. Aufnahmekatheter (100, 198) nach Anspruch 19, wobei das Betätigungsteil (338, 338B, 338C) einen Körper (340, 340B, 340C) und einen flexiblen Arm (342) aufweist, der aus dem Körper (340, 340B, 340C) nach außen vorsteht und sich zumindest teilweise durch den Schlitz (352) des Gehäuses (302) erstreckt, so dass der flexible Arm (342) derartig zugänglich ist, dass ein Benutzer das Betätigungsteil (338, 338B, 338C) entlang der Führungsbahn (324) bewegen kann.

21. Aufnahmekatheter (100, 198) nach Anspruch 14, wobei die Aufnahmehülse aufweist:
eine röhrenförmige Hülse; und
einen Verstärkungsmechanismus (150), der im Zusammenwirken mit der röhrenförmigen Hülse dafür ausgebildet bzw. vorgesehen ist, der röhrenförmigen Hülse axiale und radiale Festigkeit zu verleihen.

22. Aufnahmekatheter (100, 198) nach Anspruch 14, wobei die Aufnahmehülse (108, 204) eine röhrenförmige Hülse aufweist.

23. Aufnahmekatheter (100, 198) nach Anspruch 14, ferner mit einer Übergangshülse, die zumindest über einem Abschnitt einer Außenfläche des länglichen Teils (106, 212) und zumindest einem Abschnitt der Außenfläche der Aufnahmehülse (108, 204) angeordnet ist.

24. Aufnahmekatheter (100, 198) nach Anspruch 14, wobei das zweite Lumen (126) proximal zu einem distalen Ende des länglichen Teils (106, 212) endet.

25. Aufnahmekatheter (100, 198) nach Anspruch 14, ferner mit einer sekundären Hülse (142), die mit der Aufnahmehülse (108, 204) gekoppelt ist, wobei die sekundäre Hülse (142) sich von einer Aufnahmehülse (108, 204) zu einer Stelle erstreckt, wo das zweite Lumen endet, wenn die Aufnahmehülse (108, 204) um eine maximale Strecke in einer distalen Richtung bewegt worden ist.

26. Aufnahmekatheter (100, 198) nach Anspruch 24, wobei die sekundäre Hülle (142) das Einknicken des Entfaltungsteils (120, 200, 310) verhindert, wenn die Aufnahmehülse (108, 204) in der distalen Richtung bewegt wird.

27. Aufnahmekatheter (100, 198) nach Anspruch 14, wobei durch die Bewegung des Entfaltungsteils (120, 200, 310) die Aufnahmehülse (108, 204) relativ zu dem länglichen Teil (106, 212) bewegt wird, um mit mindestens einem Abschnitt der Embolieschutzvorrichtung in Eingriff zu treten oder diesen freizugeben.

## Revendications

1. Cathéter de capture (100, 198) capable d'être déplacé le long d'un fil guide au sein d'une lumière corporelle et de s'engager avec un dispositif de protection embolique qui collecte une matière embolique, le cathéter de capture (100, 198) comprenant :
un organe allongé (106, 212) comprenant une extrémité proximale (102) et une extrémité distale atraumatique (104, 215), l'organe allongé (106, 212) comprenant une première lumière (124) s'étendant depuis ladite extrémité proximale (102) vers ladite extrémité distale (104, 215) de celui-ci et une seconde lumière (126) s'étendant depuis ladite extrémité distale (104, 215) vers ladite extrémité proximale (102) ;
un manchon de capture (108, 204) coopérant de manière coulissante avec ladite extrémité distale (104, 215) dudit organe allongé (106, 212) ; et
un ensemble d'actionnement (110, 300, 300A, 300B, 300C) raccordé à ladite extrémité proximale (102) dudit organe allongé (106, 212), ledit ensemble d'actionnement (110, 300, 300A, 300B, 300C) comportant un organe de déploiement (120, 200, 310) qui se couple avec ledit manchon de capture (108, 204) et coopère de manière coulissante avec ladite première lumière (124) de sorte qu'un opérateur peut déplacer ledit organe de déploiement (120, 200, 310) de manière contrôlée à l'aide d'une seule main de l'opérateur ;
dans lequel l'extrémité distale atraumatique (104, 215) agit comme une transition entre un diamètre extérieur du fil guide et le cathéter de capture (100, 198) et aide à manoeuvrer et à faire naviguer le cathéter de capture (100, 198) à travers la lumière corporelle.

2. Cathéter de capture (100, 198) selon la revendication 1, dans lequel ledit ensemble d'actionnement comprend :
un cylindre (112) raccordé à ladite extrémité proximale (102) dudit organe allongé (106, 212) ; et
un piston (116, 116a) disposé de manière coulissante dans ledit cylindre (112), ledit piston (116, 116a) étant raccordé audit organe de déploiement (120, 200, 310).

3. Cathéter de capture (100, 198) selon la revendication 1, dans lequel ledit ensemble d'actionnement (110, 300, 300A, 300B, 300C) comprend :
un logement (302) ; et
un ensemble de coulissement (322) disposé dans ledit logement (302), ledit ensemble de coulissement (322) étant raccordé audit organe de déploiement (120, 200, 310) de telle sorte que ledit organe de déploiement (120, 200, 310) peut être déplacé de manière proximale et distale.

4. Cathéter de capture (100, 198) selon la revendication 3, dans lequel ledit ensemble de coulissement (322) comprend :
une fente (352) formée sur une surface dudit logement (302) ;
une piste (324) disposée dans ledit logement (302) ; et
un organe d'actionnement (338, 338B, 338C) disposé de manière coulissante sur ladite piste (324), ledit organe de déploiement (120, 200, 310) étant raccordé audit organe d'actionnement (338, 338B, 338C).

5. Cathéter de capture (100, 198) selon la revendication 4, dans lequel ledit organe d'actionnement (338, 338B, 338C) comprend un corps (340, 340B, 340C), et un bras flexible (342) dépendant vers l'extérieur depuis ledit corps (340, 340B, 340C) et s'étendant au moins partiellement à travers ladite fente (352) dudit logement (302) de telle sorte que ledit bras flexible (342) est accessible par un opérateur pour déplacer ledit organe d'actionnement (338, 338B, 338C) le long de ladite piste (324).

6. Cathéter de capture (100, 198) selon la revendication 4, dans lequel ladite fente (352) comporte au moins une rainure de verrouillage (354) formée sur sa longueur, dans lequel ledit bras flexible (342) peut être sélectivement disposé dans ladite au moins une rainure de verrouillage (354).

7. Cathéter de capture (100, 198) selon la revendication 4, comprenant en outre une pluralité de nervures tactiques formées sur une surface intérieure dudit logement (302).

8. Cathéter de capture (100, 198) selon la revendication 4, comprenant en outre un limiteur de compression (346) formé sur ledit corps (340, 340B, 340C) dudit organe d'actionnement (338, 338B, 338C).

9. Cathéter de capture (100, 198) selon la revendication 1, dans lequel ledit ensemble d'actionnement (110, 300, 300A, 300B, 300C) comprend en outre des marques sur un logement (302) dudit ensemble d'actionnement (110, 300, 300A, 300B, 300C), lesdites marques formant des indicateurs quant à l'état déployé et non déployé dudit manchon de capture (108, 204).

10. Cathéter de capture (100, 198) selon la revendication 1, dans lequel ledit ensemble d'actionnement (110, 300, 300A, 300B, 300C) comprend en outre une ou plusieurs butées (334, 336) qui limitent le mouvement dudit organe de déploiement (120, 200, 310) pendant le déploiement et la récupération du dispositif de protection embolique.

11. Cathéter de capture (100, 198) selon la revendication 1, comprenant en outre un manchon secondaire (142) couplé audit manchon de capture (108, 204), ledit manchon secondaire (142) s'étendant dans une direction proximale depuis une extrémité proximale dudit manchon de capture (108, 204).

12. Cathéter de capture (100, 198) selon la revendication 1, dans lequel ledit manchon de capture (108, 204) comprend en outre :
un manchon tubulaire configuré pour entourer au moins partiellement le dispositif de protection embolique et pour retenir le dispositif de protection embolique à l'intérieur sans perdre de quantités significatives de débris emboliques depuis celui-ci ; et
un mécanisme de renforcement (150) coopérant avec ledit manchon tubulaire pour conférer audit manchon tubulaire une résistance radiale et axiale afin d'empêcher ledit manchon tubulaire de flamber ou de s'affaisser contre le dispositif de protection embolique.

13. Cathéter de capture (100, 198) selon la revendication 1, dans lequel ledit organe de déploiement (120, 200, 310) comprend des mécanismes de renforcement aux deux extrémités pour empêcher ledit organe de déploiement (120, 200, 310) de flamber ou de s'affaisser pendant le déplacement dudit organe de déploiement (120, 200,310).

14. Cathéter de capture (100, 198) selon la revendication 1, dans lequel
la première lumière (124) est configurée pour recevoir le fil guide et la seconde lumière (126) est configurée pour recevoir l'organe de déploiement (120, 200, 310), ladite extrémité distale atraumatique (104, 215) étant adaptée pour empêcher un mouvement dudit organe allongé (106, 212) d'une extrémité distale à une extrémité proximale du dispositif de protection embolique ; et
dans lequel le manchon de capture (108, 204) coopère de manière coulissante avec au moins une partie d'une surface extérieure dudit organe allongé (106, 212).

15. Cathéter de capture (100, 198) selon la revendication 14, dans lequel ledit ensemble d'actionnement comprend :
un cylindre (112) raccordé à ladite extrémité proximale (102) dudit organe allongé (106, 212), ledit cylindre (112) comportant une chambre (115) formée à l'intérieur ; et
un piston (116, 116a) s'engageant de manière coulissante avec au moins une partie de ladite chambre (115), ledit piston (116, 116a) étant raccordé audit organe de déploiement (120, 200, 310) de telle sorte qu'un déplacement dudit piston (116, 116a) déplace ledit organe de déploiement (120, 200, 310).

16. Cathéter de capture (100, 198) selon la revendication 15, dans lequel ledit cylindre (112) est venu de matière avec ledit organe allongé (106, 212).

17. Cathéter de capture (100, 198) selon la revendication 15, dans lequel ledit piston (116, 116a) comprend un organe télescopique externe (134) et un organe télescopique interne (136) disposés de manière coulissante autour dudit organe allongé (106, 212).

18. Cathéter de capture (100, 198) selon la revendication 17, dans lequel ledit organe télescopique interne (136) est collé autour de ladite extrémité dudit organe de déploiement (120, 200, 310) pour renforcer ledit organe de déploiement (120, 200, 310).

19. Cathéter de capture (100, 198) selon la revendication 14, dans lequel ledit ensemble d'actionnement comprend :
un logement (302) ;
une fente (352) formée sur une surface du logement (302) ;
une piste (324) disposée dans le logement (302) ; et
un organe d'actionnement (338, 338B, 338C) disposé de manière coulissante sur la piste (324), ledit organe de déploiement (120, 200, 310) étant raccordé audit organe d'actionnement (338, 338B, 338C).

20. Cathéter de capture (100, 198) selon la revendication 19, dans lequel ledit organe d'actionnement (338, 338B, 338C) comprend un corps (340, 340B, 340C), et un bras flexible (342) dépendant vers l'extérieur depuis ledit corps (340, 340B, 340C) et s'étendant au moins partiellement à travers ladite fente (352) dudit logement (302) de telle sorte que ledit bras flexible (342) est accessible par un opérateur pour déplacer ledit organe d'actionnement (338, 338B, 338C) le long de ladite piste (324).

21. Cathéter de capture (100, 198) selon la revendication 14, dans lequel ledit manchon de capture comprend :
un manchon tubulaire ; et
un mécanisme de renforcement (150) disposé en coopération avec ledit manchon tubulaire pour conférer une résistance axiale et radiale audit manchon tubulaire.

22. Cathéter de capture (100, 198) selon la revendication 14, dans lequel ledit manchon de capture (108, 204) comprend un manchon tubulaire.

23. Cathéter de capture (100, 198) selon la revendication 14, comprenant en outre un manchon de transition disposé au-dessus d'au moins une partie d'une surface extérieure dudit organe allongé (106, 212) et au moins une partie de ladite surface extérieure dudit manchon de capture (108, 204).

24. Cathéter de capture (100, 198) selon la revendication 14, dans lequel ladite seconde lumière (126) se termine proximalement à une extrémité distale dudit organe allongé (106, 212).

25. Cathéter de capture (100, 198) selon la revendication 14, comprenant en outre un manchon secondaire (142) couplé audit manchon de capture (108, 204), dans lequel ledit manchon secondaire (142) s'étend depuis ledit manchon de capture (108, 204) vers un emplacement où ladite seconde lumière se termine sur ledit manchon de capture (108, 204) déplacé d'une distance maximale dans une direction distale.

26. Cathéter de capture (100, 198) selon la revendication 24, dans lequel ledit manchon secondaire (142) empêche le flambage dudit organe de déploiement (120, 200, 310) lorsque ledit manchon de capture (108, 204) est déplacé dans la direction distale.

27. Cathéter de capture (100, 198) selon la revendication 14, dans lequel un déplacement dudit organe de déploiement (120, 200, 310) déplace ledit manchon de capture (108, 204) par rapport audit organe allongé (106, 212) pour s'engager avec ou libérer au moins une partie du dispositif de protection embolique.
